# EUROPEAN PATENT APPLICATION

(11) **EP 3 747 956 A1**
(43) Date of publication of application: **09.12.2020**
(21) Application number: 19748136.9
(22) Date of filing: 31.01.2019
(51) Int. Cl.: C09B 31/062, A61K 8/49, A61Q 5/10, C09B 29/12, C09B 57/00, D06P 1/18

(54) **STAINING COMPOSITION, STAINED ARTICLE, AND AZO DYE**

(30) Priority: 31.01.2018 JP 2018014808
(71) Applicant: FUJIFILM Corporation, Tokyo 106-8620 (JP)
(72) Inventor: FUJITA, Akinori, Fujinomiya-shi, Shizuoka 418-8666 (JP); ISHIWATA, Yasuhiro, Fujinomiya-shi, Shizuoka 418-8666 (JP); JIMBO, Yoshihiro, Fujinomiya-shi, Shizuoka 418-8666 (JP)
(74) Representative: Hoffmann Eitle
(86) International application number: PCT/JP2019/003504
(87) International publication number: WO 2019/151451

(57) **Abstract**

Provided are a dyeing composition which contains an azo coloring agent represented by Formula 1 or 2, a dyed product which is obtained by being dyed with the dyeing composition, and a novel azo coloring agent. In Formulae 1 and 2, C¹ represents a heteroaromatic group, A¹ and B¹ each independently represent a heteroaromatic group, an aromatic group, or a group having a methine structure, and at least one of A¹ or B¹ represents a heteroaromatic group, D¹ represents an anthraquinonyl group, and E¹ represents a heteroaromatic group, an aromatic group, or a group having a methine structure.

A¹-N=N-C¹-N=N-B¹ Formula 1

D¹-N=N-E¹ Formula 2

## Description

### BACKGROUND OF THE INVENTION

### 1. Field of the Invention

The present disclosure relates to a dyeing composition, a dyed product, and an azo coloring agent.

### 2. Description of the Related Art

In the related art, azo coloring agents often have various kinds of visible light absorption, and thus have been used as dyes in various fields. For example, azo coloring agents have been used in various fields such as coloring of synthetic resins, printing inks, coloring agents for sublimation type thermal transfer materials, ink jet inks, and coloring agents for color filters.

In recent years, color images have become mainstream as image recording materials, and the use of coloring agents has been diversified. Specifically, coloring agents have widely been used in ink jet type recording materials, thermal transfer type recording materials, electrophotographic type recording materials, transfer type silver halide photosensitive materials, and printing inks. Further, color filters that record and reproduce color images have been used in imaging devices, for example, imaging elements such as a CCD or a CMOS and in displays such as an LCD or a PDP. In these color image recording materials and color filters, colorants (dyes or pigments) of three primary colors using a so-called additive color mixing method or subtractive color mixing method have been used in order to reproduce or record full color images.

Various dyeing agents that dye fibers and the like have been known for a long time. Further, in recent years, a plurality of hair dyeing agents that dye hair and the like have been developed.

Hair dyeing agents can be classified according to the presence or absence of a decolorizing effect on a dye or melanin to be used. Typical examples of the hair dyeing agents include a two-agent type permanent hair dyeing agent formed of a first agent that contains an alkaline agent, an oxidation dye, and optionally a direct dye such as a nitro dye, and a second agent that contains an oxidizing agent; and a one-agent type semi-permanent hair dyeing agent that contains an organic acid or an alkali agent and at least one of as an acid dye, a basic dye, or a direct dye such as a nitro dye.

Examples of azo coloring agents of the related art include those described in JP2003-342139A, JP2005-139427A, JP2001-261535A, and JP1997-508939A(JP-H09-508939A).

### SUMMARY OF THE INVENTION

An object to be achieved by an aspect of the present invention is to provide a dyeing composition with excellent dyeing properties and excellent washing resistance of a dyed product to be obtained.

An object to be achieved by another aspect of the present invention is to provide a dyed product obtained by being dyed with the dyeing composition.

An object to be achieved by still another aspect of the present invention is to provide a novel azo coloring agent.

Methods for achieving the above-described objects includes the following aspects.
<1> A dyeing composition comprising: an azo coloring agent represented by Formula 1 or 2.

   A¹-N=N-C¹-N=N-B¹ Formula 1

   D¹-N=N-E¹ Formula 2

   In Formulae 1 and 2,
   C¹ represents a heteroaromatic group,
   each of A¹ and B¹ independently represents a heteroaromatic group, an aromatic group, or a group having a methine structure, and at least one of A¹ or B¹ represents a heteroaromatic group,
   none of A¹, B¹, and C¹ has a carboxy group, a sulfo group, -SO₂F, or a cation structure,
   D¹ represents an anthraquinonyl group,
   E¹ represents a heteroaromatic group, an aromatic group, or a group having a methine structure, the heteroaromatic group as E¹ is a heteroaromatic group which has been substituted with a hydroxy group or a monosubstituted or unsubstituted amino group on a heteroaromatic ring or a heteroaromatic group which has an NH moiety in a ring structure, and the aromatic group as E¹ is an aromatic group which has been substituted with a hydroxy group or a monosubstituted or unsubstituted amino group on an aromatic ring, and
   neither D¹ nor E¹ has a carboxy group or a sulfo group.
<2> The dyeing composition according to <1>, in which C¹ in Formula 1 represents a group represented by any one of Formulae (C-1) to (C-7). 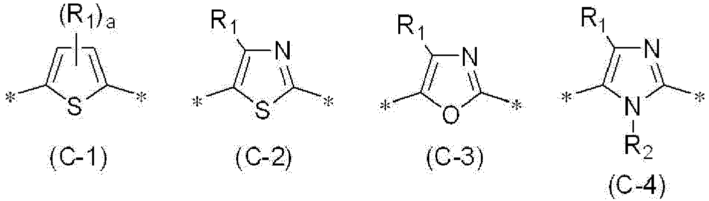 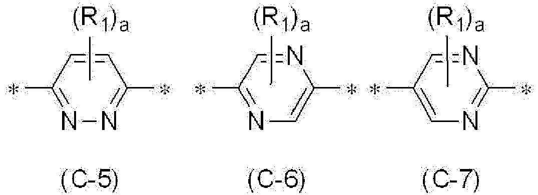 In Formulae (C-1) to (C-7),
   each R₁ independently represents a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, a represents an integer from 0 to 2,
   R₂ represents a hydrogen atom, an alkyl group, or an aryl group, and
   * represents a position bonded to an azo group in Formula 1.
<3> The dyeing composition according to <1> or <2>, wherein A¹ in Formula 1 represents an aromatic group or a group represented by any one of Formulae (A-1) to (A-25), and B¹ represents a group represented by any one of Formulae (B-1) to (B-15). 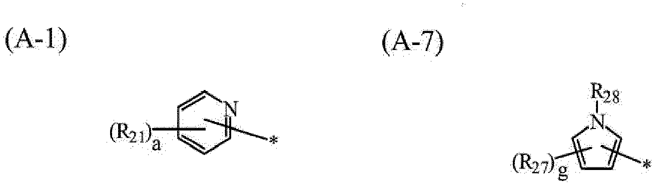 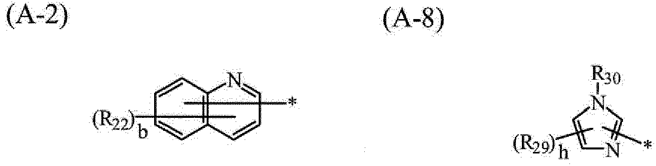 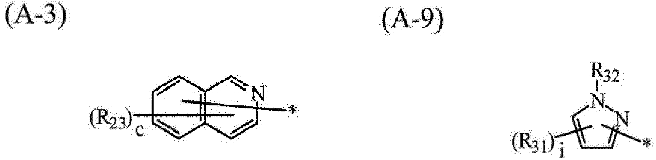 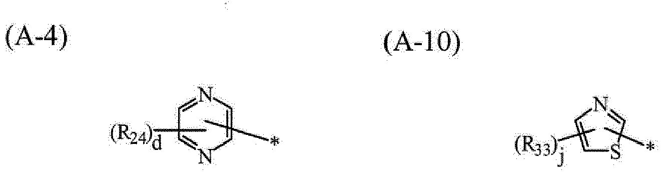 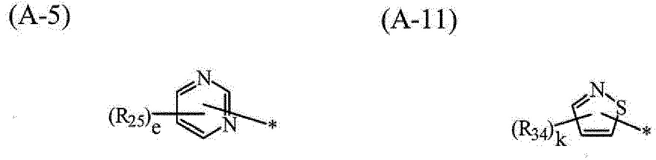 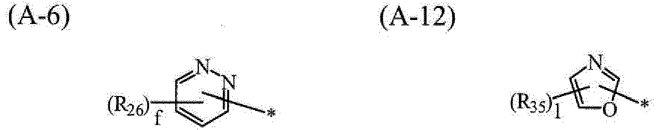 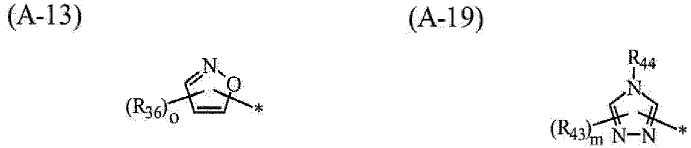 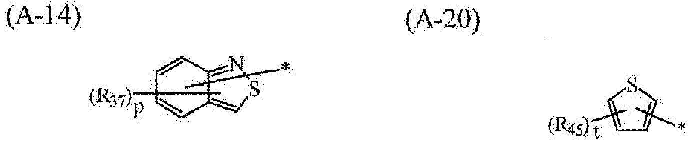 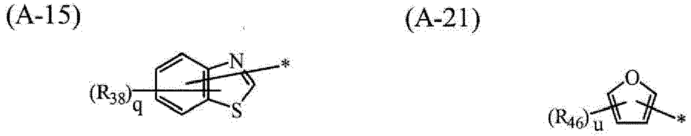 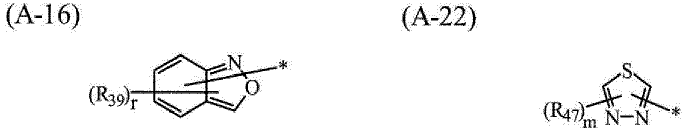 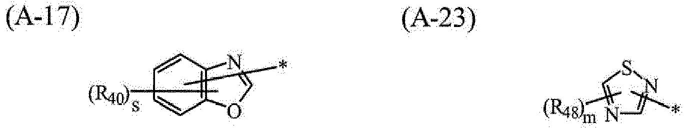 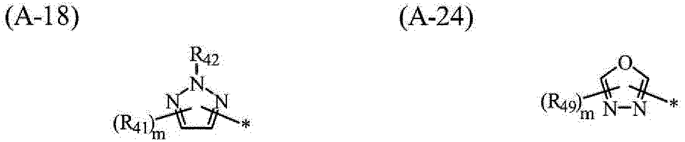 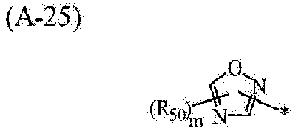 In Formulae (A-1) to (A-25),
   * represents a position bonded to an azo group in Formula 1,
   each of R₂₁ to R₅₀ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
   adjacent groups among R₂₁ to R₅₁ may be bonded to each other to form a saturated or unsaturated 5- or 6-membered ring structure,
   each of a, p, q, r, and s independently represents an integer from 0 to 4,
   each of b and c independently represents an integer from 0 to 6,
   each of d, e, f, g, t, and u independently represents an integer from 0 to 3,
   each of h, i, j, k, l, and o independently represents an integer from 0 to 2, and
   m represents 0 or 1. 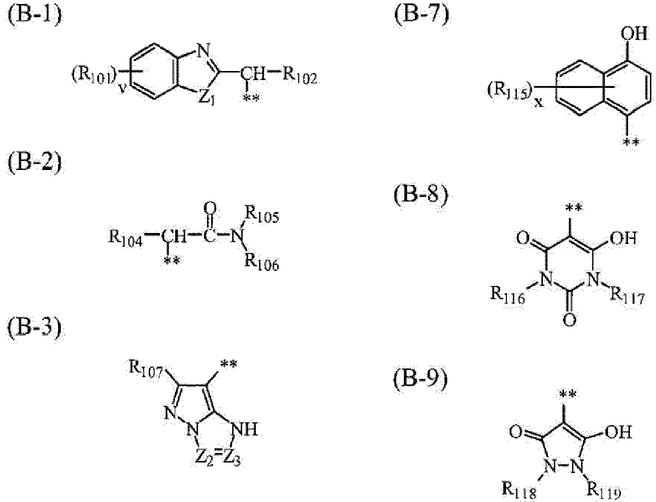 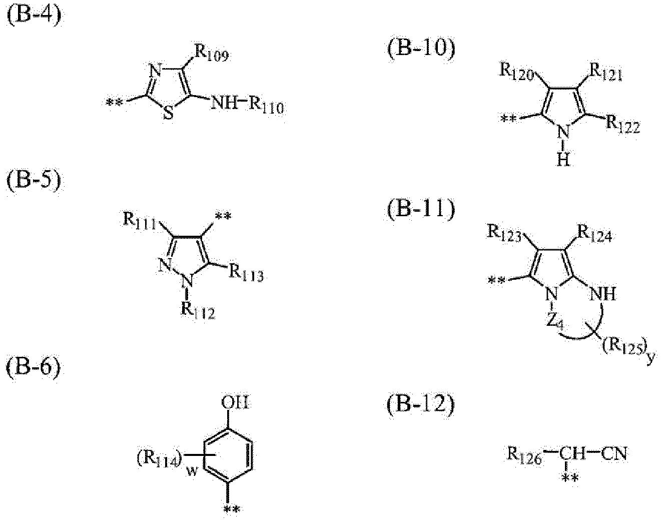 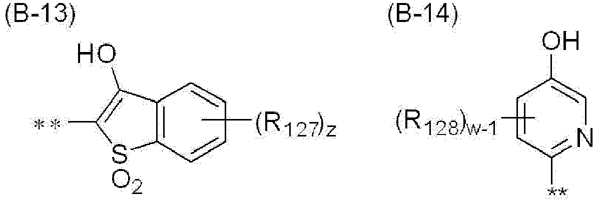 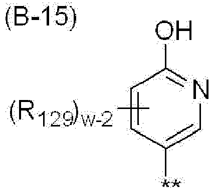
   In Formulae (B-1) to (B-15),
   ** represents a position bonded to an azo group in Formula 1,
   each R₁₀₁ independently represents a halogen atom, an alkyl group, an aryl group, a heterocyclic group, a cyano group, an alkoxy group, an amino group (including an anilino group), an acylamino group, an alkylsulfonylamino group, an arylsulfonylamino group, an alkylthio group, an arylthio group, a sulfamoyl group, an alkylsulfonyl group, or a carbamoyl group,
   v represents an integer from 0 to 4,
   each of R₁₀₂ and R₁₀₄ independently represents a cyano group, an alkylsulfonyl group, an arylsulfonyl group, an acyl group, an alkoxycarbonyl group, an aryloxycarbonyl group, or a carbamoyl group,
   Z₁ represents an oxygen atom, a sulfur atom, or -N(R₁₀₃)-,
   R₁₀₃ represents a hydrogen atom, an alkyl group, an aryl group, or a heterocyclic group,
   each of R₁₀₅ and R₁₀₆ independently represents a hydrogen atom, an alkyl group, an aryl group, or a heterocyclic group,
   R₁₀₇ represents a hydrogen atom, an alkyl group, an aryl group, a heterocyclic group, an alkoxy group, an aryloxy group, an amino group (including an anilino group), an acylamino group, an alkylsulfonylamino group, an arylsulfonylamino group, an alkylthio group, an arylthio group, an alkylsulfonyl group, an arylsulfonyl group, or a carbamoyl group,
   each of Z₂ and Z₃ independently represents -C(R₁₀₈)= or -N=,
   each R₁₀₈ independently represents an alkyl group, an aryl group, a heterocyclic group, an alkylthio group, an arylthio group, an alkoxycarbonyl group, or a carbamoyl group,
   two R₁₀₈'s may be bonded to each other to form a carbocyclic or heterocyclic ring in a case in which both Z₂ and Z₃ represent -C(R₁₀₈)=,
   R₁₀₉ represents an alkyl group, an aryl group, or a heterocyclic group,
   R₁₁₀ represents a hydrogen atom, an alkyl group, an aryl group, a heterocyclic group, an acyl group, an alkylsulfonyl group, or an arylsulfonyl group,
   R₁₁₁ represents an alkyl group, an aryl group, an alkoxy group, an amino group, an alkoxycarbonyl group, a cyano group, an acylamino group, or a carbamoyl group,
   R₁₁₂ represents a hydrogen atom, an alkyl group, an aryl group, or a heterocyclic group,
   R₁₁₃ represents a hydroxy group or an amino group,
   each of R₁₁₄ and R₁₁₅ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
   w represents an integer from 0 to 4,
   x represents an integer from 0 to 6,
   each of R₁₁₆, R₁₁₇, R₁₁₈, and R₁₁₉ independently represents an alkyl group or an aryl group,
   each of R₁₂₀ and R₁₂₁ independently represents an alkyl group, an aryl group, a heterocyclic group, a cyano group, an alkylsulfonyl group, an arylsulfonyl group, an alkoxycarbonyl group, or a carbamoyl group,
   R₁₂₂ represents a hydrogen atom, an alkyl group, an aryl group, a heterocyclic group, an acylamino group, an alkylsulfonylamino group, or an arylsulfonylamino group,
   each of R₁₂₃ and R₁₂₄ independently represents an alkyl group, an aryl group, a heterocyclic group, a cyano group, an alkylsulfonyl group, an arylsulfonyl group, an alkoxycarbonyl group, or a carbamoyl group,
   Z₄ represents a non-metallic atomic group that forms a 5- or 6-membered ring together with two nitrogen atoms and one carbon atom,
   R₁₂₅ represents an alkyl group, an aryl group, an alkoxy group, an amino group, an acylamino group, an alkylsulfonylamino group, an arylsulfonylamino group, an alkylthio group, an arylthio group, an acyl group, an alkoxycarbonyl group, or a carbamoyl group,
   y represents an integer from 0 to 2 in a case in which Z₄ forms a 5-membered ring,
   y represents an integer from 0 to 3 in a case in which Z₄ forms a 6-membered ring,
   R₁₂₆ represents an alkyl group or an aryl group,
   each R₁₂₇ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
   z represents an integer from 0 to 4,
   each of R₁₂₈ and R₁₂₉ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
   w-1 represents an integer from 0 to 3, and
   w-2 represents an integer from 0 to 3.
<4> The dyeing composition according to any one of <1> to <3>, wherein the azo coloring agent represented by Formula 1 or 2 has a molecular weight of 600 or less.
<5> The dyeing composition according to any one of <1> to <4>, wherein the azo coloring agent represented by Formula 1 or 2 has a molecular weight of 495 or less.
<6> The dyeing composition according to <3>, wherein A¹ in Formula 1 represents an aromatic group or a group represented by any one of Formulae (A-1), (A-9), (A-14) and (A-23), and B¹ represents a group represented by any one of Formulae (B-1), (B-5), (B-6), (B-7), (B-9), (B-13), (B-14), and (B-15).
<7> The dyeing composition according to <2>, wherein C¹ in Formula 1 represents a group represented by any one of Formulae (C-1) to (C-5).
<8> The dyeing composition according to any one of <1> to <7>, wherein the azo coloring agent represented by Formula 1 or 2 contains at least one group selected from the group consisting of a hydroxy group, an amino group, and a cyano group.
<9> The dyeing composition according to any one of <1> to <8>, wherein the azo coloring agent represented by Formula 1 or 2 contains a hydroxy group.
<10> The dyeing composition according to any one of <1> to <9>, comprising: the azo coloring agent represented by Formula 1.
<11> The dyeing composition according to any one of <1> to <9>, comprising: the azo coloring agent represented by Formula 2.
<12> The dyeing composition according to any one of <1> to <11>, wherein the dyeing composition is a hair dyeing composition.
<13> The dyeing composition according to <12>, wherein the dyeing composition is a hair coloring composition.
<14> A dyed product which is obtained by being dyed with the dyeing composition according to any one of <1> to <13>.
<15> An azo coloring agent which is represented by Formula 1-A.

   A^{1A}-N=N-C^{1A}-N=N-B^{1A} Formula 1-A

   In Formula 1-A,
   C^{1A} represents a group represented by any one of Formulae (C-1), (C-2), (C-3), (C-4), and (C-5),
   A^{1A} represents an aromatic group or a group represented by any one of Formulae (A-1), (A-9), (A-14), and (A-23),
   B^{1A} represents a group represented by any one of Formulae (B-5), (B-6), (B-7), (B-13), (B-14), and (B-15), and
   none of A^{1A}, B^{1A}, and C^{1A} has a carboxy group, a sulfo group, -SO₂F, or a cation structure. 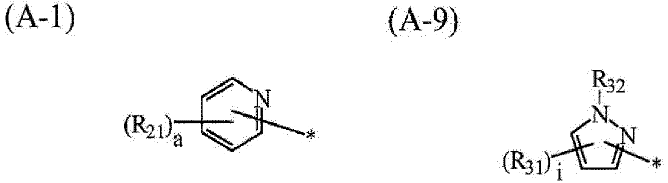 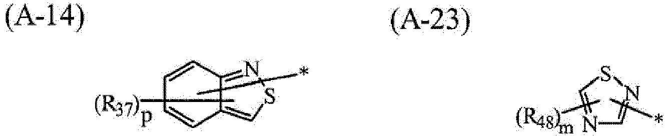
   In Formulae (A-1), (A-9), (A-14), and (A-23),
   * represents a position bonded to an azo group in Formula 1-A,
   each of R₂₁, R₃₁, R₃₂, R₃₇, and R₄₈ independently represents a hydrogen atom, a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
   adjacent groups among R₂₁, R₃₁, R₃₂, R₃₇, and R₄₈ may be bonded to each other to form a saturated or unsaturated 5- or 6-membered ring structure,
   each of a and p independently represents an integer from 0 to 4,
   i represents an integer from 0 to 2, and
   m represents 0 or 1. 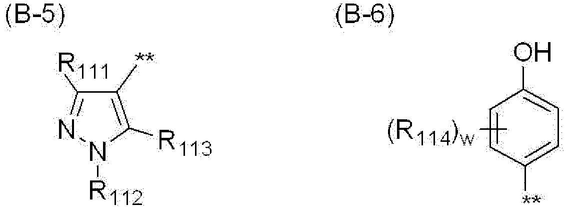 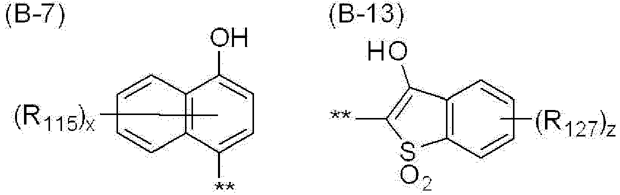 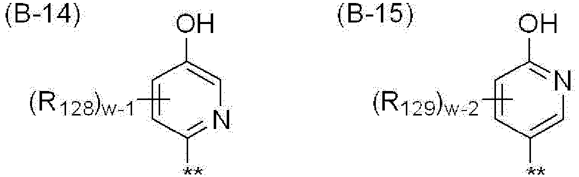
   In Formulae (B-5), (B-6), (B-7), (B-13), (B-14), and (B-15),
   ** represents a position bonded to an azo group in Formula 1-A,
   R₁₁₁ represents an alkyl group, an aryl group, an alkoxy group, an amino group, an alkoxycarbonyl group, a cyano group, an acylamino group, or a carbamoyl group,
   R₁₁₂ represents a hydrogen atom, an alkyl group, an aryl group, or a heterocyclic group,
   R₁₁₃ represents a hydroxy group or an amino group,
   each of R₁₁₄ and R₁₁₅ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
   w represents an integer from 0 to 4,
   x represents an integer from 0 to 6,
   each R₁₂₇ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
   z represents an integer from 0 to 4,
   each of R₁₂₈ and R₁₂₉ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
   w-1 represents an integer from 0 to 3, and
   w-2 represents an integer from 0 to 3. 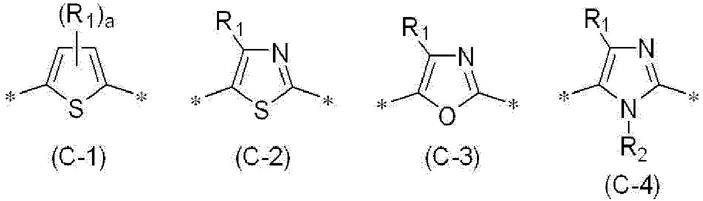 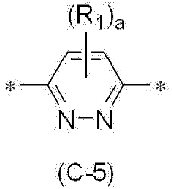
   In Formulae (C-1), (C-2), (C-3), (C-4), and (C-5),
   each R₁ independently represents a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
   a represents an integer from 0 to 2,
   R₂ represents a hydrogen atom, an alkyl group, or an aryl group, and
   * represents a position bonded to an azo group in Formula 1-A.
   R₁ may have a substituent at an optional position.
<16> An azo coloring agent which is represented by Formula 2-A.

   D^{1A}-N=N-E^{1A} Formula 2-A

In Formula 2-A, D^{1A} represents an anthraquinonyl group, and E^{1A} represents a group represented by Formula E-1.

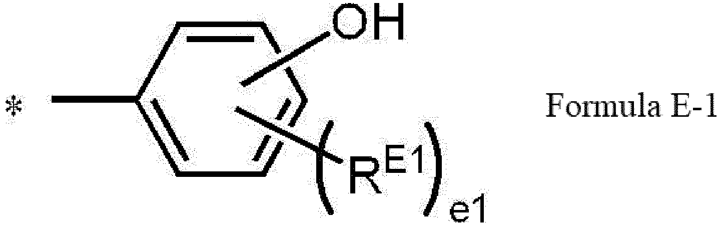

In Formula E-1,
* represents a position bonded to an azo group in Formula 2-A,
each R^{E1} independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, and
e1 represents an integer from 1 to 4.

According to an embodiment of the present invention, it is possible to provide a dyeing composition with excellent dyeing properties and excellent washing resistance of a dyed product to be obtained.

According to another embodiment of the present invention, it is possible to provide a dyed product obtained by being dyed with the dyeing composition.

According to still another embodiment of the present invention, it is possible to provide a novel azo coloring agent.

### BRIEF DESCRIPTION OF THE DRAWINGS

Fig. 1 shows an absorption spectrum of a compound 1 measured by measuring solution absorption characteristics.
Fig. 2 shows an absorption spectrum of a compound 21 measured by measuring solution absorption characteristics.
Fig. 3 shows an absorption spectrum of a compound 22 measured by measuring solution absorption characteristics.
Fig. 4 shows an absorption spectrum of a comparative compound 1 measured by measuring solution absorption characteristics.
Fig. 5 shows an absorption spectrum of a comparative compound 2 measured by measuring solution absorption characteristics.
Fig. 6 shows a reflection spectrum of a silk dyed product obtained in Example 1.
Fig. 7 shows a reflection spectrum of a silk dyed product obtained in Example 2.
Fig. 8 shows a reflection spectrum of a silk dyed product obtained in Example 3.
Fig. 9 shows a reflection spectrum of a silk dyed product obtained in Comparative Example 1.

### DESCRIPTION OF THE PREFERRED EMBODIMENTS

Hereinafter, the contents of the present disclosure will be described in detail. The description of constituent elements below is made based on representative embodiments of the present disclosure in some cases, but the present disclosure is not limited to such embodiments.

In the present specification, "to" showing a numerical range is used to mean that the numerical values described before and after "to" are included as the lower limit and the upper limit.

In a numerical range described in a stepwise manner in the present specification, an upper limit or a lower limit described in one numerical range may be replaced with an upper limit or a lower limit in another numerical range described in a stepwise manner. Further, in a numerical range described in the present specification, an upper limit or a lower limit described in the numerical range may be replaced with a value described in an example.

Further, in a case where substitution or unsubstitution is not noted in regard to the notation of a group (atomic group) in the present specification, the group includes not only a group that does not have a substituent but also a group that has a substituent. For example, the concept of an "alkyl group" includes not only an alkyl group that does not have a substituent (unsubstituted alkyl group) but also an alkyl group that has a substituent (substituted alkyl group).

In addition, the "steps" in the present specification include not only independent steps but also steps whose intended purposes are achieved even in a case where the steps cannot be precisely distinguished from other steps. Further, in the present disclosure, "% by mass" and "% by weight" have the same definition, and "part by mass" and "part by weight" have the same definition.

Further, in the present disclosure, a combination of two or more preferred aspects is a more preferred aspect.

Hereinafter, the present disclosure will be described in detail.

### (Dyeing composition)

The dyeing composition according to the embodiment of the present disclosure contains an azo coloring agent represented by Formula 1 or Formula 2.

Further, the dyeing composition according to the embodiment of the present invention can be suitably used as a hair dyeing composition because the dyeing composition has excellent dyeing properties and excellent washing resistance of a dyed product to be obtained.

A¹-N=N-C¹-N=N-B¹ Formula 1

D¹-N=N-E¹ Formula 2

In Formulae 1 and 2, C¹ represents a heteroaromatic group, A¹ and B¹ each independently represent a heteroaromatic group, an aromatic group, or a group having a methine structure, and at least one of A¹ or B¹ represents a heteroaromatic group, none of A¹, B¹, and C¹ has a carboxy group, a sulfo group, -SO₂F, or a cation structure, D¹ represents an anthraquinonyl group, E¹ represents a heteroaromatic group, an aromatic group, or a group having a methine structure, the heteroaromatic group as E¹ is a heteroaromatic group which has been substituted with a hydroxy group or a monosubstituted or unsubstituted amino group on a heteroaromatic ring or a heteroaromatic group which has an NH moiety in a ring structure, and the aromatic group as E¹ is an aromatic group which has been substituted with a hydroxy group or a monosubstituted or unsubstituted amino group on an aromatic ring, and neither D¹ nor E¹ has a carboxy group or a sulfo group.

As a result of intensive research conducted by the present inventors, it was found that a dyeing composition with excellent dyeing properties and excellent washing resistance of a dyed product to be obtained can be provided by employing the above-described configuration.

The mechanism of the excellent effects from the above-described configuration is not clear, but is assumed as follows.

The present inventors found that the balance between the dyeing properties and the washing resistance of a dyed product to be obtained is not yet sufficient in a case where a dyeing composition of the related art is used.

It is assumed that since a disazo dye or monoazo dye having a specific structure shown as the azo coloring agent represented by Formula 1 or 2 does not contain a carboxy group, a sulfo group, or a cation moiety which has a structure with high polarity, and the dye permeates into a dyed product due to the structure of a dye molecule and an appropriate molecular weight, the dyeing properties are excellent, the coloring agent is unlikely to be desorbed, and the washing resistance of a dyed product to be obtained is excellent.

Further, dyeing compositions of the related art, particularly hair dyeing compositions obtained by using direct dyes, frequently impart vivid colors and frequently have degraded versatility with respect to toning colors for black hair and brown hair. However, since the dyeing composition according to the embodiment of the present disclosure uses a disazo dye or monoazo dye which has a specific structure shown as the azo coloring agent represented by Formula 1 or 2, the brightness (for example, L in the Lab color system) and the saturation (for example, a and b in the Lab color system) are low, and dyeing of colors that are not vivid can be carried out so that a dyed product which has excellent practicality and versatility and has a dull color tone can also be prepared.

### <Azo coloring agent represented by Formula 1 or 2>

The dyeing composition according to the embodiment of the present disclosure contains an azo coloring agent represented by Formula 1 or Formula 2.

Further, it is preferable that the dyeing composition according to the embodiment of the present disclosure contains an azo coloring agent represented by Formula 1 from the viewpoint of dyeing properties and preferable that the dyeing composition contains an azo coloring agent represented by Formula 2 from the viewpoint of washing resistance of a dyed product to be obtained.

In Formula 1, none of A¹, B¹, and C¹ has a carboxy group, a sulfo group, -SO₂F, or a cation structure. Since A¹, B¹, and C¹ do not have the above-described specific structure, a dyed product to be obtained has excellent washing resistance.

Further, in Formula 2, neither D¹ nor E¹ has a carboxy group or a sulfo group. Since A1, B1, and C1 do not have the above-described specific structure, a dyed product to be obtained has excellent washing resistance.

Further, the carboxy group and sulfo group described here include neutral groups such as -COONa and -SO₃Na in addition to these acid groups.

Further, as the azo coloring agent represented by Formula 1 or 2, a compound represented by Formula 1-A or 2-A shown below is preferably exemplified from the viewpoints of the dyeing properties and washing resistance of a dyed product to be obtained.

From the viewpoints of the dyeing properties and the solubility in a case of dyeing, it is preferable that the azo coloring agent represented by Formula 1 or 2 contains at least one group selected from the group consisting of a hydroxy group, an amino group, and a cyano group, more preferable that the azo coloring agent contains at least one group selected from the group consisting of a hydroxy group and a cyano group, and particularly preferable that the azo coloring agent contains a hydroxy group.

Further, as the azo coloring agent represented by Formula 1 or 2, from the viewpoints of the dyeing properties and the washing resistance of a dyed product to be obtained, a compound having a molecular weight of 1000 or less is preferable, a compound having a molecular weight of 600 or less is more preferable, a compound having a molecular weight of 495 or less is still more preferable, and a compound having a molecular weight of 300 to 495 is particularly preferable.

In Formula 1, C¹ represents a heteroaromatic group (divalent heteroaromatic group).

The heteroaromatic ring in the heteroaromatic group is not limited as long as the heteroaromatic ring has heteroatoms such as a sulfur atom, a nitrogen atom, and an oxygen atom as a ring member. However, from the viewpoints of the dyeing properties and the color tone of a dyed product to be obtained, a heteroaromatic ring having at least one atom selected from the group consisting of a sulfur atom, a nitrogen atom, and an oxygen atom is preferable, a heteroaromatic ring having at least one atom selected from the group consisting of a sulfur atom and a nitrogen atom is more preferable, and a heteroaromatic ring having at least a sulfur atom is particularly preferable.

Further, from the viewpoints of the dyeing properties and the color tone of a dyed product to be obtained, as the heteroaromatic ring having a nitrogen atom, a heteroaromatic ring having two or more heteroatoms including nitrogen atoms is preferable, and a heteroaromatic ring having two heteroatoms including nitrogen atoms is more preferable.

From the viewpoints of the dyeing properties and the color tone of a dyed product to be obtained, it is preferable that the heteroaromatic ring in the heteroaromatic group is a 5- or 6-membered ring and more preferable that the heteroaromatic ring is a 5-membered ring.

Further, from the viewpoint of the hue, it is preferable that C¹ represents a divalent heteroaromatic group in which a heteroaromatic ring is directly bonded to each of two azo groups in Formula 1.

From the viewpoints of the dyeing properties and the hue, C¹ in Formula 1 represents preferably a group represented by any one of Formulae (C-1) to (C-7), more preferably a group represented by any one of Formulae (C-1) to (C-5), still more preferably a group represented by Formula (C-1), (C-2), or (C-4), particularly preferably a group represented by Formula (C-1) or (C-2), and most preferably a group represented by Formula (C-1).

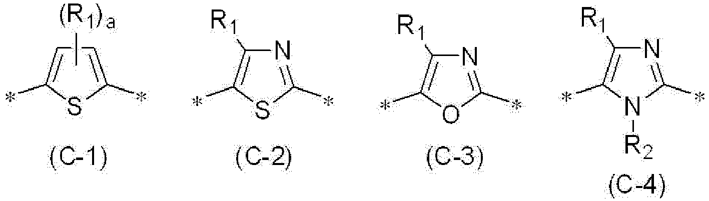

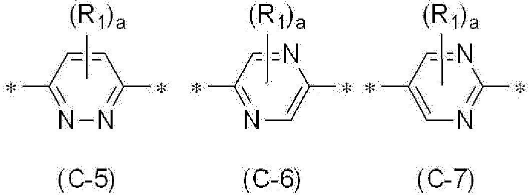

In Formulae (C-1) to (C-7), R₁'s each independently represent a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, a represents an integer of 0 to 2, R₂ represents a hydrogen atom, an alkyl group, or an aryl group, and * represents a position bonded to an azo group in Formula 1.

From the viewpoints of the dyeing properties and the color tone of a dyed product to be obtained, R₁'s in Formulae (C-1) to (C-7) each independently represent preferably a hydrogen atom, an alkyl group, an aryl group, a heterocyclic group, a cyano group, a hydroxy group, a nitro group, an alkoxy group, an aryloxy group, an amino group, or an anilino group, more preferably a hydrogen atom, an alkyl group, or an aryl group, still more preferably an alkyl group or an aryl group, and particularly preferably a methyl group or a phenyl group.

Further, from the viewpoints of the dyeing properties and the washing resistance of a dyed product to be obtained, R₁ represents preferably a group having 0 to 30 carbon atoms, more preferably a group having 1 to 20 carbon atoms, and particularly preferably a group having 1 to 8 carbon atoms.

From the viewpoints of the dyeing properties and the washing resistance of a dyed product to be obtained, a in Formulae (C-1) and (C-5) to (C-7) represents preferably 0 or 1 and more preferably 1.

In Formula (C-4), from the viewpoints of the dyeing properties and the hue, R₂ represents preferably an alkyl group or an aryl group, more preferably an alkyl group, and particularly preferably a methyl group.

A¹ and B¹ in Formula 1 each independently represent a heteroaromatic group, an aromatic group, or a group having a methine structure, and at least one of A¹ or B¹ represents a heteroaromatic group.

Further, from the viewpoints of the dyeing properties and the color tone of a dyed product to be obtained, it is preferable that A¹ and B¹ represent different groups.

Further, from the viewpoints of the dyeing properties and the color tone of a dyed product to be obtained, it is preferable that A¹ and B¹ in Formula 1 each independently represent a heteroaromatic group or an aromatic group.

The group having a methine structure in A¹ and B¹ include not only a group having a simple methine structure but also a group having a structure that is an unsaturated bond due to keto-enol tautomerism (such as a group represented by Formula (B-8) or Formula (B-9) shown below).

From the viewpoint of the dyeing properties, it is preferable that the group having a methine structure in A¹ and B¹ is a group having a methine structure that is directly bonded to an azo group in Formula 1.

Further, it is preferable that the group having a methine structure in A¹ and B¹ is a group in which one group selected from the group consisting of a heteroaromatic group and an aromatic group is bonded to a methine structure.

As the heteroaromatic group and the aromatic group in the group having a methine structure, the heteroaromatic group and the aromatic group as A¹ and B¹ are preferably exemplified.

Further, from the viewpoint of the hue of a dye, as the group having a methine structure in A¹ and B¹, a group in which a cyano group is bonded to a methine structure is preferably exemplified.

The aromatic group in A¹ and B¹ may be a ring fused with another ring.

Among the examples, from the viewpoint of the dyeing properties and the washing resistance of a dyed product to be obtained, the aromatic group as A¹ and B¹ is preferably a substituted or unsubstituted phenyl group or a substituted or unsubstituted naphthyl group and more preferably a substituted or unsubstituted phenyl group.

Examples of the substituent which may be included in the aromatic group as A¹ and B¹ include substituents described below.

Further, from the viewpoint of the dyeing properties, the aromatic group as A¹ and B¹ is preferably an aromatic group that contains at least one group selected from the group consisting of a halogen atom, a hydroxy group, a nitro group, and a cyano group and more preferably an aromatic group that contains at least one group selected from the group consisting of a halogen atom, a hydroxy group, and a nitro group.

It is preferable that the heteroaromatic group as A¹ and B¹ is a 5- or 6-membered heteroaromatic group having one or more heteroatoms selected from an oxygen atom, a sulfur atom, and a nitrogen atom in the ring.

The heteroaromatic group as A¹ and B¹ may be a ring fused with another ring. Among such fused ring groups, those fused with a 5- or 6-membered ring are preferable.

From the viewpoints of the dyeing properties and the washing resistance of a dyed product to be obtained, the number of carbon atoms in A¹ is preferably in a range of 2 to 20 and more preferably in a range of 2 to 10.

Examples of the substituent that may be included in the heteroaromatic group as A¹ and B¹ include substituents described below.

In Formula 1, the heteroaromatic group, the aromatic group, or the group having a methine structure as A¹ and B¹ may include one or more substituents, and two or more substituents may be the same as or different from one another.

Examples of the substituent include a halogen atom, an alkyl group (including a cycloalkyl group), an alkenyl group (including a cycloalkenyl group), an alkynyl group, an aryl group, a heterocyclic group, a cyano group, a hydroxy group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an anilino group), an acylamino group, an aminocarbonylamino group, an alkoxycarbonylamino group, an aryloxycarbonylamino group, a sulfamoylamino group, an alkylsulfonylamino group, an arylsulfonylamino group, a mercapto group, an alkylthio group, an arylthio group, a heterocyclic thio group, a sulfamoyl group, an alkylsulfiyl group, an arylsulfinyl group, an alkylsulfonyl group, an arylsulfonyl group, an acyl group, an alkoxycarbonyl group, an aryloxycarbonyl group, a carbamoyl group, an arylazo group, a heterocyclic azo group, an imide group, a phosphino group, a phosphinyl group, a phosphinyloxy group, a phosphinylamino group, and a silyl group. More specifically, examples of the substituent include a halogen atom (such as a chlorine atom, a bromine atom, or an iodine atom), an alkyl group (a linear group, branched, or cyclic alkyl group having 1 to 10 carbon atoms and preferably 1 to 6 carbon atoms such as methyl, ethyl, n-propyl, isopropyl, t-butyl, n-octyl-2-chloroethyl-2-cyanoethyl-2-ethylhexyl, cyclopropyl, or cyclopentyl), an alkenyl group (a linear group, branched, or cyclic alkenyl groups having 2 to 10 carbon atoms and preferably 2 to 6 carbon atoms such as vinyl, allyl, prenyl, or cyclopenten-1-yl), an alkynyl group (an alkynyl group having 2 to 10 carbon atoms and preferably 2 to 6 carbon atoms such as ethynyl or propargyl), an aryl group (an aryl group having 6 to 12 carbon atoms and preferably 6 to 8 carbon atoms such as phenyl, p-tolyl, naphthyl, or 3-chlorophenyl-2-aminophenyl), a heterocyclic group (a monovalent group having 1 to 12 carbon atoms and preferably 2 to 6 carbon atoms, which is obtained by removing one hydrogen atom from a 5- or 6-membered aromatic or non-aromatic heterocyclic compound, such as 1-pyrazolyl-1-imidazolyl-2-furyl-2-thienyl or 4-pyrimidinyl-2-benzothiazolyl), a cyano group, a hydroxyl group, a nitro group, an alkoxy group (a linear, branched, or cyclic alkoxy group having 1 to 10 carbon atoms and preferably 1 to 6 carbon atoms such as methoxy, ethoxy, isopropoxy, t-butoxy, or cyclopentyloxy-2-buten-1-yloxy-2-methoxyethoxy), an aryloxy group (an aryloxy group having 6 to 12 carbon atoms and preferably 6 to 8 carbon atoms such as phenoxy-2-methylphenoxy, 4-t-butylphenoxy, or 3-nitrophenoxy), a silyloxy group (a silyloxy group having 3 to 10 carbon atoms and preferably 3 to 6 carbon atoms such as trimethylsilyloxy or t-butyldimethylsilyloxy), a heterocyclic oxy group (a heterocyclic oxy group having 1 to 12 carbon atoms and preferably 2 to 6 carbon atoms such as 1-phenyltetrazole-5-oxy-2-tetrahydropyranyloxy), an acyloxy group (an acyloxy group having 1 to 12 carbon atoms and preferably 1 to 8 carbon atoms such as a formyloxy group, acetyloxy, pivaloyloxy, benzoyloxy, or p-methoxyphenylcarbonyloxy), a carbamoyloxy group (a carbamoyloxy group having 1 to 10 carbon atoms and preferably 1 to 6 carbon atoms such as N, N-dimethylcarbamoyloxy, N, N-diethylcarbamoyloxy, morpholinocarbonyloxy, or N, N-octylcarbamoyloxy), an alkoxycarbonyloxy group (an alkoxycarbonyloxy group having 2 to 10 carbon atoms and preferably 2 to 6 carbon atoms such as methoxycarbonyloxy, ethoxycarbonyloxy, t-butoxycarbonyloxy, or n-octyloxycarbonyloxy), an aryloxycarbonyloxy group (an aryloxycarbonyloxy group having 7 to 12 carbon atoms and preferably 7 to 10 carbon atoms such as phenoxycarbonyloxy or p-methoxyphenoxycarbonyloxy), an amino group (an amino group, an alkylamino group having 1 to 10 carbon atoms and preferably 1 to 6 carbon atoms, a dialkylamino group having 2 to 20 carbon atoms and preferably 2 to 12 carbon atoms, an anilino group having 6 to 12 carbon atoms and preferably 6 to 8 carbon atoms, or a heterocyclic amino group having 1 to 12 carbon atoms and preferably 2 to 6 carbon atoms, such as amino, methylamino, dimethylamino, anilino, N-methyl-anilino, diphenylamino, imidazol-2-ylamino, or pyrazol-3-ylamino), an acylamino group (an alkylcarbonylamino group having 1 to 10 carbon atoms and preferably 1 to 6 carbon atoms, an arylcarbonylamino group having 6 to 12 carbon atoms and preferably 6 to 8 carbon atoms, or a heterocyclic carbonylamino group having 2 to 12 carbon atoms and preferably 2 to 6 carbon atoms, such as formylamino, acetylamino, pivaloylamino, benzoylamino, pyridine-4-carbonylamino, or thiophen-2-carbonylamino), an aminocarbonylamino group (an aminocarbonylamino group having 1 to 12 carbon atoms and preferably 1 to 6 carbon atoms such as carbamoylamino, N, N-dimethylaminocarbonylamino, N, N-diethylaminocarbonylamino, or morpholin-4-ylcarbonylamino), an alkoxycarbonylamino group (an alkoxycarbonylamino group having 2 to 10 carbon atoms and preferably 2 to 6 carbon atoms such as methoxycarbonylamino, ethoxycarbonylamino, or t-butoxycarbonylamino), an aryloxycarbonylamino group (an aryloxycarbonylamino group having 7 to 12 carbon atoms and preferably 7 to 9 carbon atoms such as phenoxycarbonylamino, p-chlorophenoxycarbonylamino, or 4-methoxyphenoxycarbonylamino), a sulfamoylamino group (a sulfamoylamino group having 0 to 10 carbon atoms and preferably 0 to 6 carbon atoms such as a sulfamoylamino, N,N-dimethylaminosulfonylamino, or N-(2-hydroxyethyl) sulfamoylamino), an alkylsulfonylamino group (an alkylsulfonylamino group having 1 to 10 carbon atoms and preferably 1 to 6 carbon atoms such as methylsulfonylamino or butylsulfonylamino), an arylsulfonylamino group (an arylsulfonylamino group having 6 to 12 carbon atoms and preferably 6 to 8 carbon atoms such as phenylsulfonylamino, 2,3,5-trichlorophenylsulfonylamino group, or p-methylphenylsulfonylamino), a mercapto group, an alkylthio group (an alkylthio group having 1 to 10 carbon atoms and preferably 1 to 6 carbon atoms such as methylthio, ethylthio, or butylthio), an arylthio group (an arylthio having 6 to 12 carbon atoms and preferably 6 to 8 carbon atoms such as phenylthio, p-chlorophenylthio, or m-methoxythio), a heterocyclic thio group (a heterocyclic thio group having 2 to 10 carbon atoms and preferably 1 to 6 carbon atoms such as 2-benzothiazolylthio-1-phenyltetrazol-5-ylthio), a sulfamoyl group (a sulfamoyl group having 0 to 10 carbon atoms and preferably 0 to 6 carbon atoms such as sulfamoyl, N-ethylsulfamoyl, N,N-dimethylsulfamoyl, N-acetylsulfamoyl, or N-benzoylsulfamoyl), an alkylsulfinyl group (an alkylsulfinyl having 1 to 10 carbon atoms and preferably 1 to 6 carbon atoms such as methylsulfinyl or ethylsulfinyl), an arylsulfinyl groups (an arylsulfinyl groups having 6 to 12 carbon atoms and preferably 6 to 8 carbon atoms such as phenylsulfinyl or p-methylphenylsulfinyl), an alkylsulfonyl group (an alkylsulfonyl group having 1 to 10 carbon atoms and preferably 1 to 6 carbon atoms such as methylsulfonyl or ethylsulfonyl), an arylsulfonyl group (an arylsulfonyl group having 6 to 12 carbon atoms and preferably 6 to 8 carbon atoms such as phenyl sulfonyl or p-chlorophenylsulfonyl), an acyl group (a formyl group, an alkylcarbonyl group having 2 to 10 carbon atoms and preferably 2 to 6 carbon atoms, or an arylcarbonyl group having 7 to 12 carbon atoms and preferably 7 to 9 carbon atoms, such as acetyl, pivaloyl-2-chloroacetyl, benzoyl, or 2,4-dichlorobenzoyl), an alkoxycarbonyl group (an alkoxycarbonyl group having 2 to 10 carbon atoms and preferably 2 to 6 carbon atoms such as methoxycarbonyl, ethoxycarbonyl, t-butoxycarbonyl, or isobutyloxycarbonyl), an aryloxycarbonyl group (an aryloxycarbonyl group having 7 to 12 carbon atoms and preferably 7 to 9 carbon atoms such as phenoxycarbonyl-2-chlorophenoxycarbonyl, 3-nitrophenoxycarbonyl, or 4-t-butylphenoxycarbonyl), a carbamoyl group (a carbamoyl group having 1 to 10 carbon atoms and preferably 1 to 6 carbon atoms such as carbamoyl, N-methylcarbamoyl, N,N-dimethylcarbamoyl, or N-(2-hydroxyethyl)carbamoyl, or N-(methylsulfonyl)carbamoyl), an arylazo group (an arylazo group having 6 to 12 carbon atoms and preferably 6 to 8 carbon atoms such as phenylazo or p-chlorophenylazo), a heterocyclic azo group (a heterocyclic azo group having 1 to 10 carbon atoms and preferably 1 to 6 carbon atoms such as pyrazol-3-ylazo or thiazol-2-ylazo-5-ethylthio-1,3,4-thiadiazol-2-ylazo), an imide group (an imide group having 2 to 10 carbon atoms and preferably 4 to 8 carbon atoms such as N-succinimide or N-phthalimide), a phosphino group (a phosphino group having 2 to 12 carbon atoms and preferably 2 to 6 carbon atoms such as dimethylphosphino, diphenylphosphino, or methylphenoxyphosphino), a phosphinyl group (a phosphinyl group having 2 to 12 carbon atoms and preferably 2 to 6 carbon atoms such as phosphinyl or diethoxyphosphinyl), a phosphinyloxy group (a phosphinyloxy group having 2 to 12 carbon atoms and preferably 2 to 6 carbon atoms such as diphenoxyphosphinyloxy or dibutoxyphosphinyloxy), a phosphinylamino group (a phosphinylamino group having 2 to 12 carbon atoms and preferably 2 to 6 carbon atoms such as dimethoxyphosphinylamino or dimethylaminophosphinylamino), and a silyl group (a silyl group having 3 to 12 carbon atoms and preferably 3 to 8 carbon atoms such as trimethylsilyl, t-butyldimethylsilyl, or phenyldimethylsilyl). In a case where these groups are groups that can be further substituted, these groups can further contain a substituent, and preferred examples of the substituent includes groups having the same definition as that for the substituent described above. In a case where these groups are substituted with two or more substituents, these substituents may be the same as or different from one another.

Particularly preferably, from the viewpoints of the dyeing properties and the washing resistance of a dyed product to be obtained, as the substituent of the heteroaromatic group, the aromatic group, or the group having a methine structure as A¹ and B¹, a halogen atom, an alkyl group, an aryl group, a heterocyclic group, a cyano group, a hydroxy group, a nitro group, an alkoxy group, an aryloxy group, an amino group (including an anilino group), an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group is preferable, and a halogen atom, an alkyl group, an aryl group, a heterocyclic group, a cyano group, a hydroxy group, a nitro group, an alkoxy group, an amino group (including an anilino group), an acylamino group, an alkylsulfonylamino group, an alkylthio group, an arylthio group, a heterocyclic thio group, or a carbamoyl group is more preferable.

From the viewpoints of the dyeing properties and the washing resistance of a dyed product to be obtained, A¹ in Formula 1 represents preferably an aromatic group or a group represented by any one of Formulae (A-1) to (A-25), more preferably an aromatic group or a group represented by any one of Formulae (A-1), (A-9), (A-14), and (A-23), still more preferably an aromatic group that contains a nitro group or a group represented by any one of Formulae (A-1), (A-9), (A-14), and (A-23), and particularly preferably a group represented by any one of Formulae (A-1), (A-9), (A-14), and (A-23).

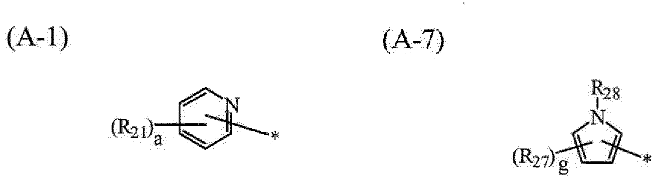

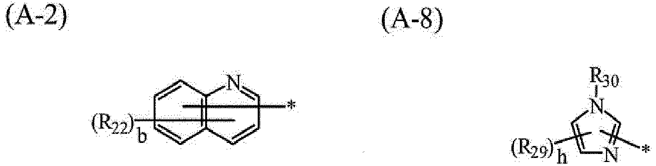

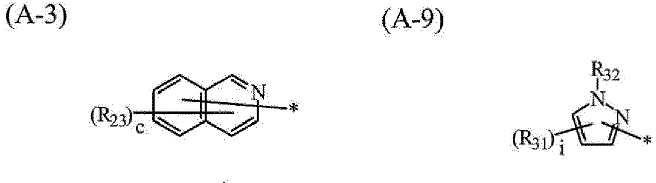

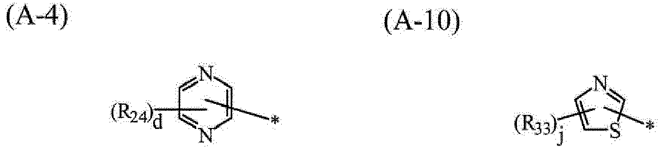

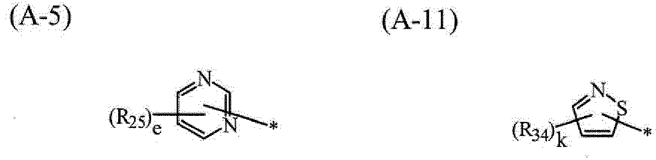

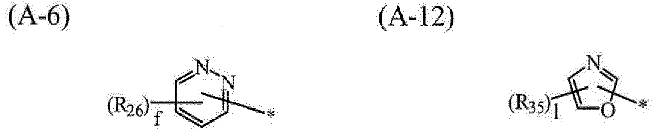

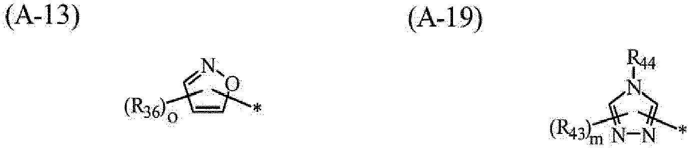

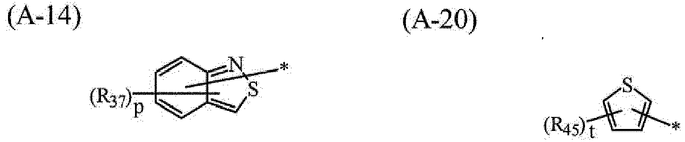

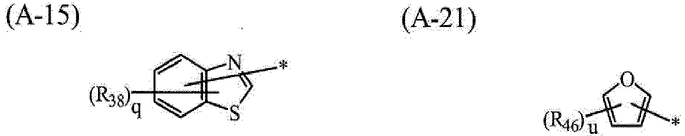

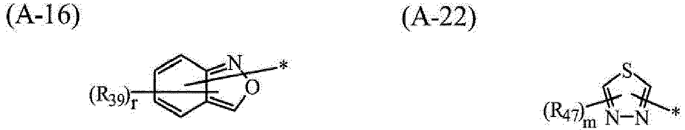

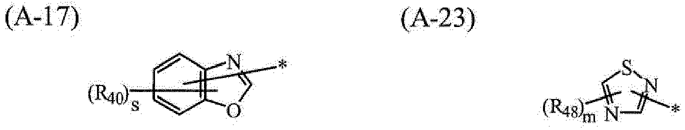

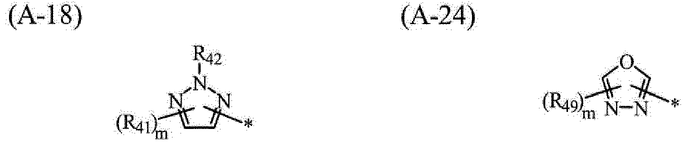

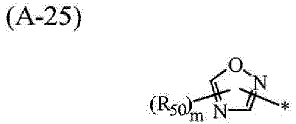

In Formulae (A-1) to (A-25), * represents a position bonded to an azo group in Formula 1,
R₂₁ to R₅₀ each independently represent a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
adjacent groups among R₂₁ to R₅₀ may be bonded to each other to form a saturated or unsaturated 5- or 6-membered ring structure,
a, p, q, r, and s each independently represent an integer of 0 to 4,
b and c each independently represent an integer of 0 to 6,
d, e, f, g, t, and u each independently represent an integer of 0 to 3,
h, i, j, k, l, and o each independently represent an integer of 0 to 2, and
m represents 0 or 1.

From the viewpoint of the dyeing properties, R₂₁ to R₅₀ each independently represent preferably a halogen atom, an alkyl group, a cyano group, a hydroxy group, a nitro group, an alkoxy group, an amino group (including an anilino group), an acylamino group, an alkylsulfonylamino group, an alkylthio group, an arylthio group, a heterocyclic thio group, or a carbamoyl group and more preferably an alkyl group, an alkylsulfonylamino group, an alkylthio group, an arylthio group, or a heterocyclic thio group.
a, p, q, r, s, b, c, d, e, f, g, t, and u each independently represent preferably an integer of 0 to 2 and more preferably 0 or 1.

It is preferable that h, i, j, k, l, and o each independently represent 0 or 1.

It is preferable that B¹ in Formula 1 represents a group derived from a coupler component. Here, the coupler component indicates a partial structure derived from a coupler compound capable of reacting with a diazonium salt to obtain an azo dye.

B¹ in Formula 1 has preferably 3 to 30 carbon atoms, more preferably 3 to 20 carbon atoms, and still more preferably 3 to 12 carbon atoms.

As the coupler component, residues of couplers known in the field of silver halide color photographic sensitive materials are preferable, and the skeleton portion (a portion which is a chromophore of a coloring agent formed by being coupled with an oxidation product of an aromatic amine developing agent such as p-phenylenediamine) of the silver halide color photographic coupler specifically described in p. 80 to 85 and 87 to 89 of Research Disclosure 37038 (February, 1995) can be used.

Preferred examples of couplers known as yellow image forming couplers in the field of silver halide color photographic sensitive materials include coupler skeletons such as pivaloylacetamide type couplers, benzoylacetamide type couplers, malon diester type couplers, malondiamide type couplers, dibenzoylmethane type couplers, benzothiazolylacetamide type couplers, malonester monoamide type couplers, benzoxazolylacetamide type couplers, benzimidazolylacetamide type couplers, cyanoacetamide type couplers, cycloalkylcarbonylacetamide type couplers, indoline-2-ylacetamide type couplers, quinazolin-4-one-2-ylacetamide type couplers described in US5021332A, benzo-1,2,4-thiadiazine-1,1-dioxide-3-ylacetamide type couplers described in US5021330A, couplers described in EP0421221A, couplers described in US5455149A, couplers described in EP0622673A, couplers described in EP0953871A, and 3-indoloylacetamide-type couplers described in EP0953871A, EP0953872A, and EP0953873A.

Preferred examples of couplers known as magenta image forming couplers in the field of silver halide color photographic sensitive materials include coupler skeletons such as 5-pyrazolone type couplers, 1H-pyrazolo[1,5-a]benzimidazole type couplers, 1H-pyrazolo[5,1-c][1,2,4]triazole type couplers, 1H-pyrazolo[1,5-b][1,2,4]triazole type couplers, 1H-imidazo[1,2-b]pyrazole type couplers, cyanoacetophenone type couplers, activated propene type couplers described in WO93/01523A, enamine type couplers and 1H-imidazo[1,2-b][1,2,4]triazole type couplers described in WO93/07534A, and couplers described in US4871652A.

Preferred examples of couplers known as cyan image forming couplers in the field of silver halide color photographic sensitive materials include coupler skeletons such as phenol type couplers, naphthol type couplers, 2,5-diphenylimidazole type couplers, 1H-pyrrolo[1,2-b][1,2,4]triazole type couplers, and 1H-pyrrolo[2,1-c][1,2,4]triazole type couplers described in EP0249453A, pyrrole type couplers described in JP1992-188137A (JP-H04-188137A) and JP1992-190347A (JP-H04-190347A), 3-hydroxypyridine type couplers described in JP1989-315736A (JP-H01-315736A), pyrrolopyrazole type couplers described in US5164289A, pyrroloimidazole type couplers described in JP1992-174429A (JP-H04-174429A), pyrazolopyrimidine type couplers described in US4950585A, pyrrolotriazine type couplers described in JP1992-204730A (JP-H04-204730A), couplers described in US4746602A, couplers described in US5104783A, couplers described in US5162196A, and couplers described in EP0556700B.

From the viewpoints of the dyeing properties and the washing resistance of a dyed product to be obtained, B¹ in Formula 1 represents preferably a group represented by any one of Formulae (B-1) to (B-15), more preferably a group represented by any one of Formulae (B-1), (B-5), (B-6), (B-7), (B-9), (B-13), (B-14), and (B-15), and particularly preferably a group represented by Formula (B-6).

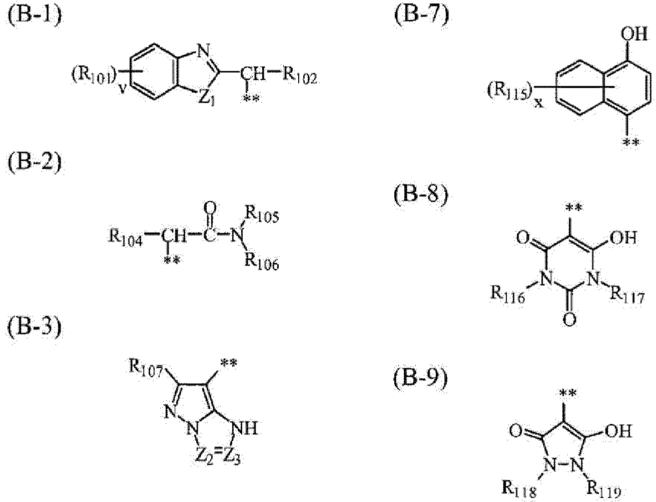

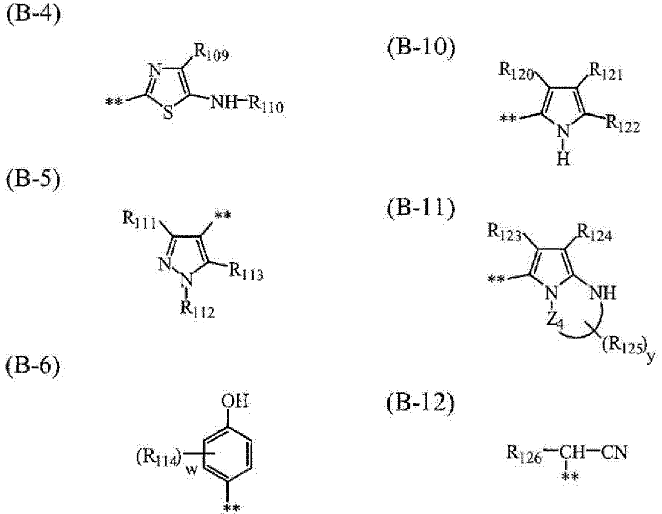

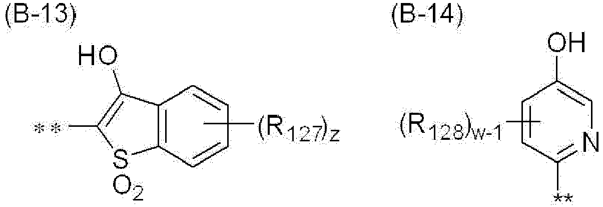

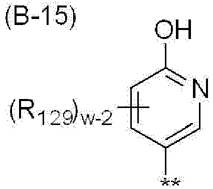

In Formulae (B-1) to (B-15),
** represents a position bonded to an azo group in Formula 1,
R₁₀₁'s each independently represent a halogen atom, an alkyl group, an aryl group, a heterocyclic group, a cyano group, an alkoxy group, an amino group (including an anilino group), an acylamino group, an alkylsulfonylamino group, an arylsulfonylamino group, an alkylthio group, an arylthio group, a sulfamoyl group, an alkylsulfonyl group, or a carbamoyl group,
v represents an integer of 0 to 4,
R₁₀₂ and R₁₀₄ each independently represent a cyano group, an alkylsulfonyl group, an arylsulfonyl group, an acyl group, an alkoxycarbonyl group, an aryloxycarbonyl group, or a carbamoyl group,
Z₁ represents an oxygen atom, a sulfur atom, or -N(R₁₀₃)-,
R₁₀₃ represents a hydrogen atom, an alkyl group, an aryl group, or a heterocyclic group,
R₁₀₅ and R₁₀₆ each independently represent a hydrogen atom, an alkyl group, an aryl group, or a heterocyclic group,
R₁₀₇ represents a hydrogen atom, an alkyl group, an aryl group, a heterocyclic group, an alkoxy group, an aryloxy group, an amino group (including an anilino group), an acylamino group, an alkylsulfonylamino group, an arylsulfonylamino group, an alkylthio group, an arylthio group, an alkylsulfonyl group, an arylsulfonyl group, or a carbamoyl group,
Z₂ and Z₃ each independently represent -C(R₁₀₈)= or -N=,
R₁₀₈'s each independently represent an alkyl group, an aryl group, a heterocyclic group, an alkylthio group, an arylthio group, an alkoxycarbonyl group, or a carbamoyl group,
two R₁₀₈'s may be bonded to each other to form a carbocyclic or heterocyclic ring in a case where both Z₂ and Z₃ represent -C(R₁₀₈)=,
R₁₀₉ represents an alkyl group, an aryl group, or a heterocyclic group,
R₁₁₀ represents a hydrogen atom, an alkyl group, an aryl group, a heterocyclic group, an acyl group, an alkylsulfonyl group, or an arylsulfonyl group,
R₁₁₁ represents an alkyl group, an aryl group, an alkoxy group, an amino group (including an anilino group), an alkoxycarbonyl group, a cyano group, an acylamino group, or a carbamoyl group,
R₁₁₂ represents a hydrogen atom, an alkyl group, an aryl group, or a heterocyclic group,
R₁₁₃ represents a hydroxy group or an amino group,
R₁₁₄ and R₁₁₅ each independently represent a halogen atom, an alkyl group, an aryl group, a heterocyclic group, a nitro group, an alkoxy group, an aryloxy group, an amino group (including an anilino group), an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w represents an integer of 0 to 4,
x represents an integer of 0 to 6,
R₁₁₆, R₁₁₇, R₁₁₈, and R₁₁₉ each independently represent an alkyl group or an aryl group,
R₁₂₀ and R₁₂₁ each independently represent an alkyl group, an aryl group, a heterocyclic group, a cyano group, an alkylsulfonyl group, an arylsulfonyl group, an alkoxycarbonyl group, or a carbamoyl group,
R₁₂₂ represents a hydrogen atom, an alkyl group, an aryl group, a heterocyclic group, an acylamino group, an alkylsulfonylamino group, or an arylsulfonylamino group,
R₁₂₃ and R₁₂₄ each independently represent an alkyl group, an aryl group, a heterocyclic group, a cyano group, an alkylsulfonyl group, an arylsulfonyl group, an alkoxycarbonyl group, or a carbamoyl group,
Z₄ represents a non-metallic atomic group that forms a 5- or 6-membered ring along with two nitrogen atoms and one carbon atom,
R₁₂₅ represents an alkyl group, an aryl group, an alkoxy group, an amino group, an acylamino group, an alkylsulfonylamino group, an arylsulfonylamino group, an alkylthio group, an arylthio group, an acyl group, an alkoxycarbonyl group, or a carbamoyl group,
y represents an integer of 0 to 2 in a case where Z₄ forms a 5-membered ring,
y represents an integer of 0 to 3 in a case where Z₄ forms a 6-membered ring,
R₁₂₆ represents an alkyl group or an aryl group,
R₁₂₇'s each independently represent a halogen atom, an alkyl group, an aryl group, a heterocyclic group, a cyano group, a hydroxy group, a nitro group, an alkoxy group, an aryloxy group, an amino group (including an anilino group), an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
z represents an integer of 0 to 4,
R₁₂₈ and R₁₂₉ each independently represent a halogen atom, an alkyl group, an aryl group, a heterocyclic group, a nitro group, an alkoxy group, an aryloxy group, an amino group (including an anilino group), an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w-1 represents an integer of 0 to 3, and
w-2 represents an integer of 0 to 3.

In Formula (B-1), it is preferable that R₁₀₁ represents a halogen atom, an acylamino group, an alkylsulfonylamino group, a sulfamoyl group, or a carbamoyl group, R₁₀₂ represents a cyano group or a carbamoyl group, and Z₁ represents an oxygen atom, a sulfur atom, or -N(R₁₀₃)- in which R₁₀₃ represents an alkyl group. v represents an integer of 0 to 4, and in a case where v represents 1 to 4, the groups as R₁₀₁ may be the same as or different from one another.

In Formula (B-2), it is preferable that R₁₀₄ represents a cyano group, an acyl group, or a carbamoyl group and one of R₁₀₅ and R₁₀₆ represents a hydrogen atom.

In Formula (B-3), R₁₀₇ represents preferably a hydrogen atom, an alkyl group, or an aryl group and more preferably a hydrogen atom or an alkyl group. R₁₀₈ represents preferably an alkyl group, an aryl group, or a heterocyclic ring and more preferably an alkyl group.

In Formula (B-4), it is preferable that R₁₀₉ represents an alkyl group or an aryl group and R₁₁₀ represents a hydrogen atom or an alkyl group.

In Formula (B-5), it is preferable that R₁₁₁ represents an alkoxycarbonyl group, a cyano group, or a carbamoyl group, R₁₁₂ represents an alkyl group or an aryl group, and R₁₁₃ represents a hydroxy group.

In Formula (B-6), R₁₁₄ represents preferably a halogen atom, an alkyl group, an acylamino group, an alkoxycarbonylamino group, an aminocarbonylamino group, an alkylsulfonylamino group, an arylsulfonylamino group, or a carbamoyl group and more preferably a halogen atom, an alkyl group, an acylamino group, or an alkylsulfonylamino group. w represents an integer of 0 to 4, and in a case where w represents 2 to 4, the groups as R₁₁₄ may be the same as or different from one another.

In Formula (B-7), R₁₁₅ represents preferably a halogen atom, an acylamino group, an alkoxycarbonylamino group, an aminocarbonylamino group, an alkylsulfonylamino group, an arylsulfonylamino group, or a carbamoyl group and more preferably an acylamino group, an alkylsulfonylamino group, or a carbamoyl group. x represents an integer of 0 to 6, and in a case where x represents 2 to 6, the groups as R₁₁₅ may be the same as or different from one another.

In Formulae (B-8) and (B-9), it is preferable that R₁₁₆ and R₁₁₇ have the same definition and R₁₁₈ and R₁₁₉ have the same definition.

In Formula (B-10), it is preferable that R₁₂₀ and R₁₂₁ represent an alkyl group, an aryl group, a heterocyclic group, or a cyano group and R₁₂₂ represents a hydrogen atom, an alkyl group, an aryl group, an acylamino group, or an alkylsulfonylamino group.

In Formula (B-11), it is preferable that R₁₂₃ represents an alkyl group, an aryl group, a heterocyclic group, or a cyano group, R₁₂₄ represents an alkylsulfonyl group, an arylsulfonyl group, an alkoxycarbonyl group, or a carbamoyl group, and R₁₂₅ represents an alkyl group, an aryl group, an alkylthio group, an amino group, or an acylamino group. y represents an integer of 0 to 2 in a case where Z₄ forms a 5-membered ring, and y represents an integer of 0 to 3 in a case where Z₄ forms a 6-membered ring.

In Formula (B-12), it is preferable that R₁₂₆ represents an alkyl group or an aryl group.

In Formula (B-13), it is preferable that R₁₂₇'s each independently represent a halogen atom, an alkyl group, an aryl group, a heterocyclic group, an acylamino group, an alkylsulfonylamino group, a sulfamoyl group, or a carbamoyl group and z represents an integer of 0 to 2.

In Formula (B-14), R₁₂₈ represents preferably a halogen atom, an alkyl group, an acylamino group, an alkoxycarbonylamino group, an aminocarbonylamino group, an alkylsulfonylamino group, an arylsulfonylamino group, or a carbamoyl group and more preferably a halogen atom, an alkyl group, an acylamino group, or an alkylsulfonylamino group. w-1 represents an integer of 0 to 3, and in a case where w-1 represents 2 or 3, the groups as R₁₂₈ may be the same as or different from one another.

In Formula (B-15), R₁₂₉ represents preferably a halogen atom, an alkyl group, an acylamino group, an alkoxycarbonylamino group, an aminocarbonylamino group, an alkylsulfonylamino group, an arylsulfonylamino group, or a carbamoyl group and more preferably a halogen atom, an alkyl group, an acylamino group, or an alkylsulfonylamino group. w-2 represents an integer of 0 to 3, and in a case where w-2 represents 2 or 3, the groups as R129 may be the same as or different from one another.

In Formulae (B-1) to (B-15), specific examples of the preferable numbers of carbon atoms and the individual groups exemplified in the description of the groups represented by R₁₀₁ to R₁₂₆ are the same as those exemplified in the description of the substituents as A¹ in Formula 1.

In a case where R₁₀₁ to R₁₂₉ in Formulae (B-1) to (B-15) represent groups that can be further substituted, R₁₀₁ to R₁₂₉ can further have a substituent. In this case, examples of the substituent are the same as those exemplified as the substituent represented by A¹ in Formula 1.

D¹ in Formula 2 represents an anthraquinonyl group and may be a 1-anthraquinonyl group or a 2-anthraquinonyl group, but it is preferable that D¹ represents a 1-anthraquinonyl group from the viewpoints of the dyeing properties and the color tone of a dyed product to be obtained.

Further, the anthraquinonyl group as D¹ may have a substituent, but an anthraquinonyl group that does not have a substituent is preferable from the viewpoint of the washing resistance of a dyed product to be obtained.

Preferred examples of the substituent which may be included in the anthraquinonyl group as D¹ include the substituents as A¹ in Formula 1.

E¹ in Formula 2 represents a heteroaromatic group, an aromatic group, or a group having a methine structure, the heteroaromatic group as E¹ is a heteroaromatic group which has been substituted with a hydroxy group or a monosubstituted or unsubstituted amino group on a heteroaromatic ring or a heteroaromatic group which has an NH moiety in a ring structure, and the amino group as E¹ is an aromatic group which has been substituted with a hydroxy group or a monosubstituted or unsubstituted amino group on an aromatic ring.

From the viewpoint of the dyeing properties, as the group having a methine structure as E¹, a group having a methine structure that is directly bonded to an azo group in Formula 2 is preferable.

Further, it is preferable that the group having a methine structure as E¹ is a group in which one group selected from the group consisting of a heteroaromatic group and an aromatic group is bonded to the methine structure.

Preferred examples of the heteroaromatic group and the aromatic group in the group having a methine structure include the heteroaromatic group and the aromatic group as E¹.

The aromatic group as E¹ may be a ring fused with another ring.

Among the examples, from the viewpoints of dyeing properties and washing resistance of a dyed product to be obtained, the aromatic group as E¹ is preferably an aromatic group substituted with a hydroxy group and more preferably a phenyl group substituted with a hydroxy group.

The aromatic group as E¹ may have a substituent other than the hydroxy group or amino group described above. Examples of the substituent that may be included in the aromatic group as E¹ include the substituent as A¹ in Formula 1.

It is preferable that the heteroaromatic group as E¹ is preferably a 5- or 6-membered heteroaromatic group having one or more heteroatoms selected from the group consisting of an oxygen atom, a sulfur atom, and a nitrogen atom in the ring.

The heteroaromatic group as E¹ may be a ring fused with another ring. Among such fused ring groups, those fused with a 5- or 6-membered ring are preferable.

The number of carbon atoms in the heteroaromatic group as E¹ is preferably in a range of 2 to 20 and more preferably in a range of 2 to 10.

Among the examples, from the viewpoints of the dyeing properties and the washing resistance of a dyed product to be obtained, E1 in Formula 2 represents preferably an aromatic group which has been substituted with a hydroxy group or a monosubstituted or unsubstituted amino group on an aromatic ring, more preferably an aromatic group which has been substituted with a hydroxy group, and particularly preferably a group represented by Formula E-1.

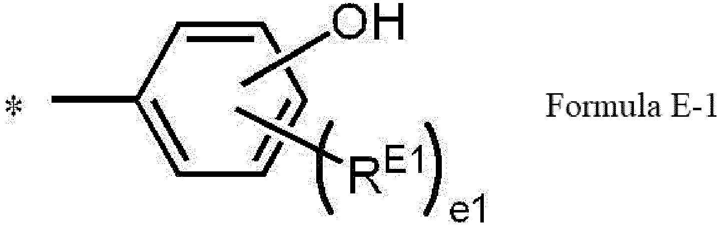

In Formula E-1, * represents a position bonded to an azo group in Formula 2, R^{E1}'s each independently represent a halogen atom, an alkyl group, an aryl group, a heterocyclic group, a cyano group, a hydroxy group, a nitro group, an alkoxy group, an aryloxy group, an amino group (including an anilino group), an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, and e1 represents an integer of 1 to 4.

From the viewpoints of the dyeing properties and the washing resistance of a dyed product to be obtained, R^{E1}'s in Formula E-1 each independently represent preferably a halogen atom, an alkyl group, an aryl group, a heterocyclic group, a cyano group, a hydroxy group, or a nitro group, more preferably a halogen atom, a cyano group, or a nitro group, still more preferably a halogen atom, and particularly preferably a chlorine atom.

From the viewpoints of the dyeing properties and the washing resistance of a dyed product to be obtained, e1 in Formula E-1 represents preferably 1 or 2 and more preferably 1.

Specific preferred examples of the azo coloring agent represented by Formula 1 include a compound in which A¹ represents a group represented by any one of Formulae 1-A to 6-A, a compound in which B¹ represents a group represented by any one of Formulae 1-B to 7-B, and a compound in which C¹ represents a group represented by any one of Formulae 1-C to 12-C, and more preferred examples thereof include compounds 1 to 20. Here, Me represents a methyl group, and * represents a bonding position with respect to an azo group. Further, "1-A" and the like in Table 1 respectively represent "Formula 1-A" and the like.

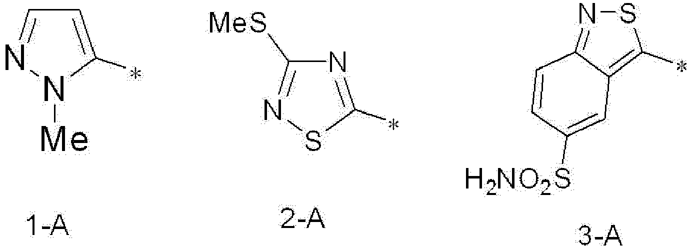

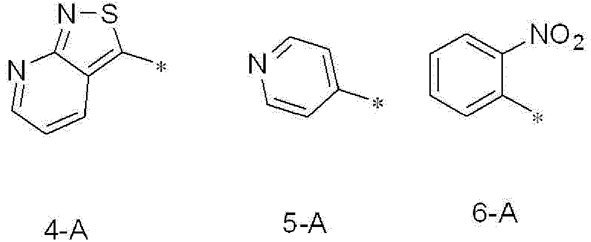

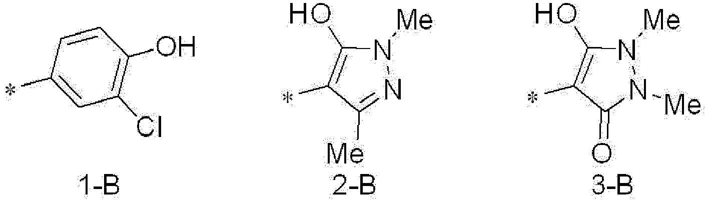

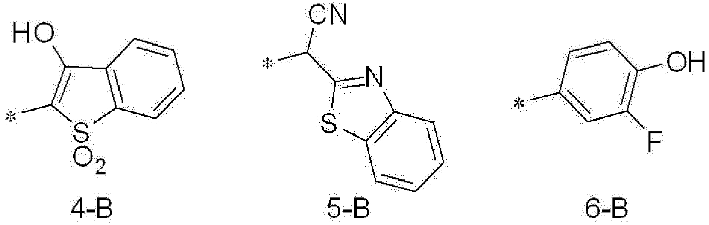

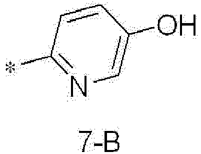

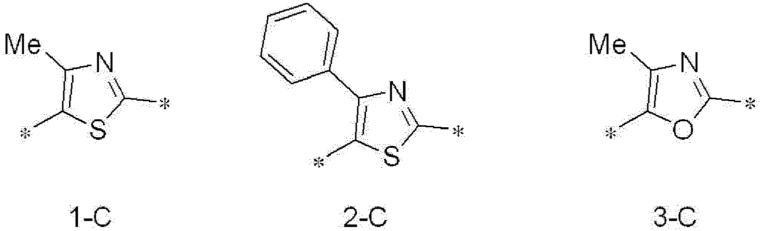

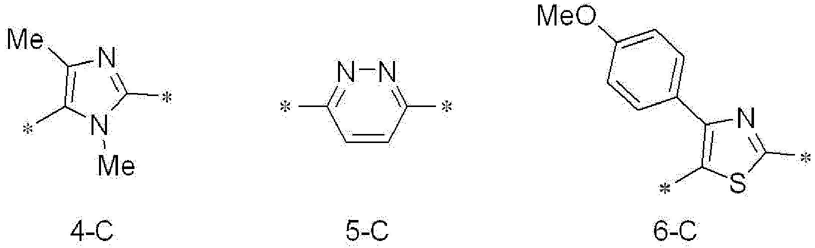

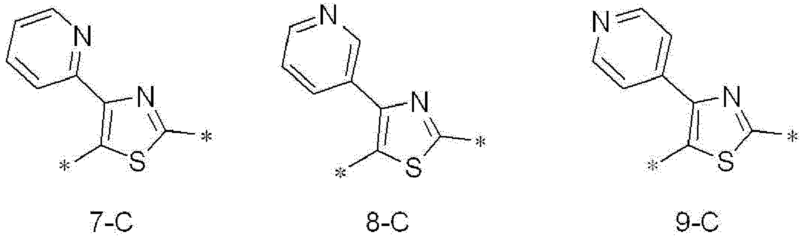

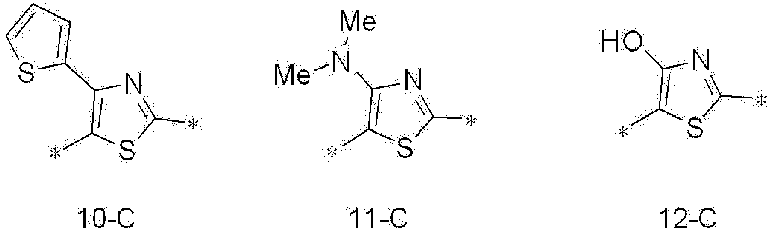

**[Table 1]**

| | A¹ | C¹ | B¹ | Molecular weight |
|---|---|---|---|---|
| Compound 1 | 1-A | 1-C | 1-B | 362 |
| Compound 2 | 2-A | 1-C | 1-B | 412 |
| Compound 3 | 3-A | 1-C | 1-B | 494 |
| Compound 4 | 4-A | 1-C | 1-B | 416 |
| Compound 5 | 5-A | 1-C | 1-B | 359 |
| Compound 6 | 6-A | 1-C | 1-B | 403 |
| Compound 7 | 1-A | 1-C | 2-B | 345 |
| Compound 8 | 1-A | 1-C | 4-B | 415 |
| Compound 9 | 1-A | 1-C | 6-B | 328 |
| Compound 10 | 1-A | 1-C | 7-B | 328 |
| Compound 11 | 1-A | 2-C | 1-B | 424 |
| Compound 12 | 1-A | 8-C | 1-B | 425 |
| Compound 13 | 1-A | 10-C | 1-B | 430 |
| Compound 14 | 1-A | 11-C | 1-B | 391 |
| Compound 15 | 1-A | 12-C | 1-B | 364 |
| Compound 16 | 1-A | 11-C | 7-B | 357 |
| Compound 17 | 1-A | 11-C | 6-B | 357 |
| Compound 18 | 1-A | 12-C | 7-B | 330 |
| Compound 19 | 2-A | 12-C | 6-B | 380 |
| Compound 20 | 2-A | 12-C | 7-B | 380 |

Specific preferred examples of the azo coloring agent represented by Formula 2 include the following compounds 21 and 22.

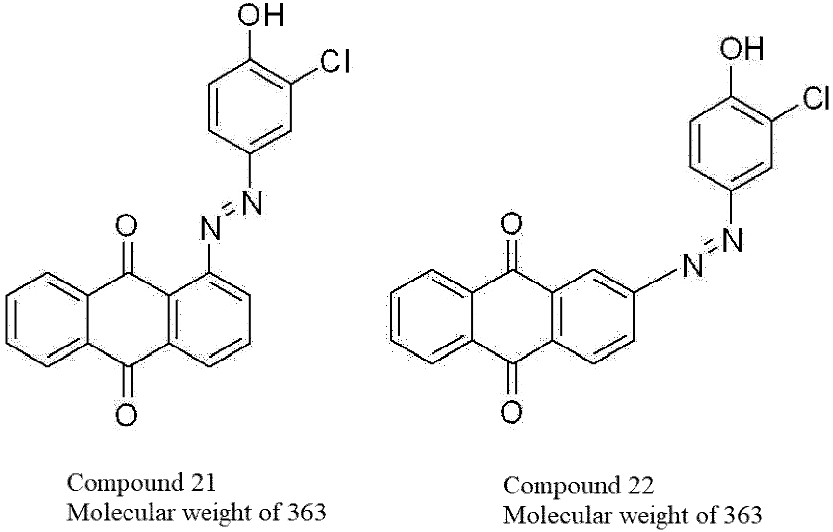

The azo coloring agent represented by Formula 1 or 2 may be used alone or in combination of two or more kinds thereof. Further, the azo coloring agent represented by Formula 1 and the azo coloring agent represented by Formula 2 may be used in combination.

The content of the azo coloring agent represented by Formula 1 or Formula 2 in the dyeing composition according to the embodiment of the present disclosure is not particularly limited. However, from the viewpoint of the dyeing properties, the content thereof is preferably in a range of 0.0001% by mass to 20% by mass, more preferably in a range of 0.001% by mass to 20% by mass, still more preferably in a range of 0.05% by mass to 10% by mass, and particularly preferably in a range of 0.1% by mass to 5% by mass with respect to the total mass of the dyeing composition.

In a case where other dyes described below are used in combination, it is preferable that the content of the other dyes is set to be smaller than the content of the azo coloring agent represented by Formula 1 or 2 from the viewpoints of the dyeing properties and the washing resistance of a dyed product to be obtained.

Further, the azo coloring agent represented by Formula 1 or 2 may be used in combination with other dyes (for example, direct dyes or oxidation dyes).

Examples of other direct dyes include Basic Blue 7 (C. I. 42595), Basic Blue 26 (C. I. 44045), Basic Blue 99 (C.I. 56059), Basic Violet 10 (C. I. 45170), Basic Violet 14 (C. I. 42515), Basic Brown 16 (C. I. 12250), Basic Brown 17 (C. I. 12251), Basic Red 2 (C. I. 50240), Basic Red 12 (C. I. CI 48070), Basic Red 22 (C. I. 11055), Basic Red 46 (C. I. 110825), Basic Red 76 (C. I. 12245), Basic Red 118 (C. I. 12251: 1), Basic Yellow 28 (C. I. 48054), and Basic Yellow 57 (C. I. 12719); cationic dyes described in JP-1983-002204A (JP-S58-002204A), JP1997-118832A (JP-H09-118832A), JP-1996-501322A (JP-H08-501322A), and JP-1996-507545A (JP-H08-507545A); and methine type cationic dyes having a cyanine structure described in paragraph 0082 of JP4080947B.

The dyeing composition according to the embodiment of the present disclosure can also be used in combination with an oxidation dye together with the azo coloring agent represented by Formula 1 or 2. With such a combination, dyeing that cannot be obtained by using the oxidation dye alone can be performed.

As the oxidation dye, known color developing materials and coupling materials which have been commonly used for oxidation hair dyeing agent are used.

Examples of the color developing material include paraphenylenediamine, toluene-2,5-diamine, 2-chloro-paraphenylenediamine, N-methoxyethylparaphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3 -bis(N-(2-hydroxyethyl)-N-(4-aminophenyl)amino)-2-propanol, PEG-3,2,2'-paraphenylenediamine, paraaminophenol, paramethylaminophenol, 3-methyl-4-aminophenol, 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, and 4,5-diamino-1- (4'-chlorobenzyl)pyrazole, and salts thereof.

Examples of the coupling material include metaphenylenediamine, 2,4-diaminophenoxyethanol, 2-amino-4-(2-hydroxyethylamino) anisole, 2,4-diamino-5-methylphenetol, 2,4-diamino-5-(2-hydroxyethoxy) toluene, 2,4-dimethoxy-1,3-diaminobenzene, 2,6-bis(2-hydroxyethylamino) toluene, 2,4-diamino-5-fluorotoluene, 1,3-bis(2,4-diaminophenoxy) propane, metaaminophenol, 2-methyl-5-aminophenol, 2-methyl-5-(2-hydroxyethylamino) phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino) phenol, 2-methyl-4-fluoro-5-aminophenol, resorcinol, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1- (2-hidroxyethyl)amino-3,4-methylenedioxybenzene, 2,6-dihydroxy-3,4-dimethylpyridine, 2,6-dimethoxy-3,5-diaminopyridine, 2,3 -diamino-6-methoxypyridine, 2-methylamino-3 -amino-6-methoxypyridine, 2-amino-3-hydroxypyridine, and 2,6-diaminopyridine, and salts thereof.

These color developing materials and coupling materials may be respectively used in combination of two or more kinds thereof, and the contents thereof are respectively preferably in a range of 0.01% by mass to 20% by mass and more preferably in a range of 0.5% by mass to 10% by mass with respect to the content of the entire composition (after respective agents are mixed in a case of the two-agent type or three-agent type, the same applies hereinafter).

The dyeing composition according to the embodiment of the present disclosure may further contain autoxidation dyes represented by indoles or indolines; and known direct dyes such as nitro dyes or dispersed dyes.

The total content of the compound represented by Formula 1 or 2 and other dyes is preferably in a range of 0.001% by mass to 20% by mass, more preferably in a range of 0.01% by mass to 20% by mass, and particularly preferably in a range of 0.5% to 15% by mass with respect to the total mass of the dyeing composition.

Further, the dyeing composition according to the embodiment of the present disclosure may contain an oxidizing agent as necessary.

Examples of the oxidizing agent include hydrogen peroxide; a persulfate such as ammonium persulfate, potassium persulfate, or sodium persulfate; a perborate such as sodium perborate; a percarbonate such as sodium percarbonate; and a bromate such as sodium bromate or potassium bromate. Among these, from the viewpoints of decolorization for hair and the dyeing properties, hydrogen peroxide is particularly preferable. In addition, hydrogen peroxide can also be used in combination with other oxidizing agents. The content of the oxidizing agent is preferably in a range of 0.5% by mass to 10% by mass and more preferably in a range of 1% by mass to 8% by mass with respect to the total mass of the dyeing composition.

Further, an embodiment in which the dyeing composition according to the embodiment of the present disclosure and a composition containing an oxidizing agent are mixed and used before dyeing is also preferably exemplified.

In a case where the dyeing composition according to the embodiment of the present disclosure is used as a hair dyeing composition, it is preferable that the dyeing composition contains conditioning components suitable for application to hair. The conditioning components are typically polymers or oils that can be dissolved or dispersed in a hair dyeing agent composition and adhere to hair at the time of rinse or dilution with water or shampoo.

In a case where the conditioning components are used, the amount thereof to be blended is preferably in a range of 0.01% by mass to 30% by mass, more preferably in a range of 0.1% by mass to 20% by mass, and particularly preferably in a range of 0.1% by mass to 10% by mass with respect to the total mass of the dyeing composition.

Suitable examples of the conditioning components include cationic polymers, silicones, conditioning agents characterized as organic conditioning oils (such as hydrocarbon oil, polyolefin, and fatty acid ester), and combinations thereof. In addition to these, conditioning agents that form dispersion liquid particles in a surfactant aqueous solution may be exemplified.

The cationic polymer indicates a polymer containing a cationic group or a group that can be ionized to a cationic group and includes an amphoteric polymer which is cationic as a whole. That is, examples of the cationic polymer include aqueous solutions that contain an amino group or an ammonium group in a side chain of a polymer chain or contain a diallyl quaternary ammonium salt as a constitutional unit, such as cationized cellulose derivatives, cationic starch, cationized guar gum derivatives, polymers or copolymers of diallyl quaternary ammonium salts, and quaternized polyvinylpyrrolidone derivatives. Among these, from the viewpoints of softness particularly at the time of shampooing, smoothness and finger passableness, easiness of settling into shape at the time of drying and the moisturizing effect, and the stability of the agent, polymers containing diallyl quaternary ammonium salts as a constitutional unit, quaternized polyvinylpyrrolidone derivatives, and cationized cellulose derivatives are preferable, and polymers or copolymers of diallyl quaternary ammonium salts and cationized cellulose derivatives are more preferable.

Specific examples of the polymers or copolymers of diallyl quaternary ammonium salts include a dimethyldiallylammonium chloride polymer (polyquaternium-6, such as MERQUAT 100; manufactured by Ondeo Nalco Co., Ltd.), a dimethyldiallylammonium chloride/acrylic acid copolymer (polyquaternium-22, such as MERQUAT 280 and MERQUAT 295, manufactured by Ondeo Nalco Co., Ltd.), and a dimethyldiallylammonium chloride/acrylic acid amide copolymer (polyquaternium-7, such as MERQUAT 550; manufactured by Ondeo Nalco Co., Ltd.).

Specific examples of the quaternized polyvinylpyrrolidone derivative include quaternary ammonium salts (polyquaternium-11, such as GAFQUAT 734, GAFQUAT 755, and GAFQUAT 755N (all manufactured by ISP Japan Ltd.) obtained from a copolymer of vinylpyrrolidone (VP) and dimethylaminoethyl methacrylate and diethyl sulfate.

Specific examples of the cationized cellulose derivative include polymers (polyquaternium-10, such as LEOGARD G and LEOGARD GP (both manufactured by Lion Specialty Chemicals Co., Ltd.), POLYMER JR -125, POLYMER JR-400, POLYMER JR-30M, POLYMER LR-400, and POLYMER LR-30M (all manufactured by Amerchol Corporation)) of quaternary ammonium salts obtained by adding glycidyltrimethylammonium chloride to hydroxyethylcellulose; and hydroxyethylcellulose/dimethyldiallylammonium chloride copolymers (polyquaternium-4, such as CELQUAT H-100 and CELQUAT L-200 (both manufactured by National Starch and Chemical Corporation).

The cationic polymers may be used in combination of two or more kinds thereof.

From the viewpoints of the stability, the viscosity, and feeling improvement, the content of the cationic polymer is preferably in a range of 0.001% by mass to 20% by mass, more preferably in a range of 0.01% by mass to 10% by mass, and particularly preferably in a range of 0.05% by mass to 5% by mass with respect to the total mass of the dyeing composition.

It is preferable that the dyeing composition according to the embodiment of the present disclosure contains silicones in order to impart an excellent feeling of use. Examples of the silicones include polyalkoxysilanes and modified silicones (such as amino-modified silicone, fluorine-modified silicone, alcohol-modified silicone, polyether-modified silicone, epoxy-modified silicone, and alkyl-modified silicone). Among these, polyalkoxysilanes, polyether-modified silicone, and amino-modified silicone are preferable.

The polyalkoxysilanes may be cyclic or acyclic dimethylsiloxane polymers, and examples thereof include SH200 Series, BY22-019, BY22-020, BY11-026, BY22-029, BY22-034, BY22-050A, BY22-055, BY22-060, BY22-083, and FZ-4188 (all manufactured by Dow Corning Toray Co., Ltd.), KF-9008, KM-900 Series, MK-15H, and MK-88 (all manufactured by Shin-Etsu Chemical Co., Ltd.).

As the polyether-modified silicone, silicones containing a polyoxyalkylene group may be used, and examples of the group constituting the polyoxyalkylene group include an oxyethylene group and an oxypropylene group. More specific examples thereof include KF-6015, KF-945A, KF-6005, KF-6009, KF-6013, KF-6019, KF-6029, KF-6017, KF-6043, KF-353A, KF- 354A, and KF-355A(all manufactured by Shin-Etsu Chemical Co., Ltd.), FZ-2404, SS-2805, FZ-2411, FZ-2412, SH3771M, SH3772M, SH3773M, SH3775M, SH3749, SS-280X Series, BY22- 008M, BY11-030, and BY25-337 (all manufactured by Dow Corning Toray Co., Ltd.).

As the amino-modified silicone, silicones containing an amino group or an ammonium group may be used, and examples thereof include amino-modified silicone oil in which all or some of terminal hydroxyl groups are blocked by a methyl group or the like, and amodimethicone in which the terminal is not blocked.

Specific examples of suitable commercially available products of the amino-modified silicone include amino-modified silicone oils such as SF8452C and SS-3551 (both manufactured by Dow Corning Toray Co., Ltd.), KF-8004, KF-867S, and KF-8015 (all manufactured by Shin-Etsu Chemical Co., Ltd.); and amodimethicone emulsions such as SM8704C, SM8904, BY22-079, FZ-4671, and FZ-4672 (all manufactured by Dow Corning Toray Co., Ltd.).

From the viewpoints of sufficient effects and suppression of stickiness, the total content of these silicones is preferably in a range of 0.02% by mass to 40% by mass, more preferably in a range of 0.1% by mass to 20% by mass, and particularly preferably in a range of 0.2% by mass to 15% by mass with respect to the total mass of the dyeing composition.

In a case where the dyeing composition contains the silicones and the cationic polymer, the mass ratio of the cationic polymer to the silicones in the dyeing composition is preferably in a range of 50:1 to 1:50 and more preferably in a range of 50:1 to 1:10.

From the viewpoints of feeling improvement and the stability, it is preferable that the dyeing composition according to the embodiment of the present disclosure contains a higher alcohol. In a case where the dyeing composition contains a higher alcohol, effects of forming a structure with a surfactant to prevent separation of the hair dyeing agent composition and improving the feeling at the time of rinse.

As the higher alcohol, a higher alcohol having 8 to 22 carbon atoms is preferable, and a higher alcohol having 16 to 22 carbon atoms is more preferable. Specific examples include cetyl alcohol, stearyl alcohol, and behenyl alcohol, and mixtures thereof.

The higher alcohols may be used in combination of two or more kinds thereof.

The content of the higher alcohol is preferably in a range of 0.01% by mass to 20% by mass and more preferably 0.1% by mass to 10% by mass with respect to the total mass of the dyeing composition.

The dyeing composition according to the embodiment of the present disclosure may contain a surfactant.

As the surfactant, any of a cationic surfactant, a nonionic surfactant, an amphoteric surfactant and an anionic surfactant can be used.

As the cationic surfactant, a mono-long chain alkyl quaternary ammonium salt is preferable, and specific examples thereof include cetyltrimethylammonium chloride, stearyltrimethylammonium chloride, aralkyltrimethylammonium chloride, and behenyltrimethylammonium chloride.

Examples of the nonionic surfactant include polyoxyalkylene alkyl ether, polyoxyalkylene alkenyl ether, higher fatty acid sucrose ester, polyglycerin fatty acid ester, higher fatty acid mono- or diethanolamide, polyoxyethylene hardened castor oil, polyoxyethylene sorbitan fatty acid ester, polyoxyethylene sorbitol fatty acid ester, alkyl saccharide-based surfactants, alkylamine oxide, and alkylamidoamine oxide.

Examples of the amphoteric surfactant include imidazoline-based surfactants, carbobetaine-based surfactants, amidobetaine-based surfactants, sulfobetaine-based surfactants, hydroxysulfobetaine-based surfactants, and amidosulfobetaine-based surfactants.

Examples of the anionic surfactant include an alkyl benzene sulfonate, an alkyl or alkenyl ether sulfate, an alkyl or alkenyl sulfate, an olefin sulfonate, an alkane sulfonate, a saturated or unsaturated fatty acid salt, an alkyl or alkenyl ether carboxylate, an α-sulfone fatty acid salt, a N-acylamino acid type surfactant, a phosphoric acid mono- or diester type surfactant, and a sulfosuccinate. Examples of the alkyl ether sulfate include a polyoxyethylene alkyl ether sulfate. Examples of the counter ions of anionic residues of these surfactants include an alkali metal ion such as a sodium ion or a potassium ion; an alkaline earth metal ion such as a calcium ion or a magnesium ion; an ammonium ion; an alkanolamine having one to three alkanol groups with 2 or 3 carbon atoms (for example, monoethanolamine, diethanolamine, triethanolamine, or triisopropanolamine).

The surfactants can be used alone or in combination of two or more kinds thereof.

The content of the surfactant is not particularly limited, but is preferably in a range of 0.1% by mass to 20% by mass, more preferably in a range of 0.1% by mass to 18% by mass, and still more preferably in a range of 0.5% by mass to 15% by mass with respect to the total mass of the dyeing composition.

It is preferable that the dyeing composition according to the embodiment of the present disclosure contains at least one solvent selected from the group consisting of water and an organic solvent, as a medium.

Examples of the organic solvent include lower aliphatic alcohols such as ethanol, propanol, and isopropanol; aromatic alcohols such as benzyl alcohol and benzyloxyethanol; polyols such as propylene glycol, 1,3-butanediol, diethylene glycol, and glycerin; cellosolves such as ethyl cellosolve and butyl cellosolve; and carbitols such as ethyl carbitol and butyl carbitol.

The total content of water and the organic solvent is not particularly limited and can be appropriately selected depending on the applications, but is preferably in a range of 10% by mass to 99.99% by mass, more preferably 50% by mass to 99.9% by mass, and particularly preferably in a range of 80% by mass to 99% by mass.

From the viewpoints of the dyeing properties and the solubility of the azo dye, it is preferable that the dyeing composition according to the embodiment of the present disclosure contains a basic compound.

Examples of the basic compound include ammonia; alkanolamines such as monoethanolamine, isopropanolamine, and salts thereof; guanidinium salts such as a guanidine carbonate; and hydroxides such as sodium hydroxide.

The content of the basic compound is preferably in a range of 0.01% by mass to 20% by mass, more preferably in a range of 0.1% by mass to 10% by mass, and still more preferably in a range of 0.5% by mass to 5% by mass with respect to the total mass of the dyeing composition.

In addition to the above components, other known components can be added to the dyeing composition according to the embodiment of the present disclosure. Examples of such optional components include hydrocarbons, animal and vegetable fats and oils, higher fatty acids, natural or synthetic polymers, ethers, protein derivatives, hydrolysis proteins, amino acids, preservatives, chelating agents, stabilizers, antioxidants, vegetable extracts, crude drug extracts, vitamins, flavors, ultraviolet absorbing agents, and emulsifiers.

Further, other dyes, conditioning components, surfactants, solvents, and other components can refer to the description of JP4080947B.

The dyeing composition according to the embodiment of the present disclosure may be used as a one-agent type dyeing composition, a two-agent type dyeing composition having a first agent that contains an alkaline agent and a second agent that contains an oxidizing agent, or a three-agent type dyeing composition having a powdery oxidizing agent such as a persulfate in addition to these two agents. The azo coloring agent represented by Formula 1 or Formula 2 may be incorporated into one or both of the above-described agents in the two-agent type or three-agent type dyeing composition.

For example, in a case where the dyeing composition is used as a hair dyeing composition, the hair dyeing composition is applied directly to hair in a case of using a one-agent type dyeing composition, but it is preferable that the hair dyeing composition is applied to hair after these components are mixed at the time of dyeing hair in a case of using a two-agent type or three-agent type dyeing composition.

Further, the form of the dyeing composition according to the embodiment of the present disclosure may be appropriately selected depending on the applications. For example, the dyeing composition can be made in the form of powder, a transparent liquid, an emulsion, cream, a gel, a paste, an aerosol, or aerosol foam.

In a case where the dyeing composition according to the embodiment of the present disclosure is used as a hair dyeing composition, the viscosity thereof at a stage of applying the composition to hair (after mixing in a case of a two-agent type or three-agent type) is preferably in a range of 1000 mPa·s to 100000 mPa·s, more preferably in a range of 5000 mPa·s to 50000 mPa·s, and particularly preferably in a range of 10000 mPa·s to 40000 mPa·s. Here, the viscosity is a value measured at 20°C using a Brookfield rotational viscometer (No. 5, spindle, 5 rpm).

The material to be dyed (dyed material) using the dyeing composition according to the embodiment of the present disclosure is not particularly limited as long as fibers, cloth, leather, paper, and films can be dyed. Further, the material is not particularly limited as long as dyeing can be carried out, and hair is preferably exemplified. That is, the dyeing composition according to the embodiment of the present disclosure is preferably a hair dyeing composition and more preferably a hair coloring composition.

Examples of the hair include animal fibers and human hair, and the hair may be fibrous or in the form of a sheet such as woven fabric or nonwoven fabric.

In addition, the animal fibers in the present disclosure include not only animal hair such as wool and goat hair, but also fibers obtained by modifying animal hair and silk.

Further, the dyeing composition according to the embodiment of the present disclosure can be suitably used for dyeing vegetable fibers or chemical fibers.

Further, the dyeing composition according to the embodiment of the present disclosure can be suitably used as a medical dyeing composition.

The dyeing method carried out using the dyeing composition according to the embodiment of the present disclosure is not particularly limited except that the dyeing composition according to the embodiment of the present disclosure is brought into contact with a material to be dyed, and examples thereof include a method of coating a material to be dyed with the dyeing composition according to the embodiment of the present disclosure and a method of immersing a material to be dyed in the dyeing composition according to the embodiment of the present disclosure.

In addition, the material may be heated at the time of being dyed. The heating temperature is not particularly limited, but is preferably in a range of 25°C to 80°C and more preferably in a range of 30°C to 60°C from the viewpoints of the dyeing properties and the dyeing speed.

The above-described dyeing may be performed only once or may be repeatedly performed twice or more times.

Further, after the material is dyed, it is preferable to wash the material as necessary and more preferable to wash the material with water.

### (Dyed product)

The dyed product according to the embodiment of the present disclosure is dyed by the dyeing composition according to the embodiment of the present disclosure.

The dyed product according to the embodiment of the present disclosure has excellent washing resistance and can be suitably used for applications where washing is performed after repeated use.

The shape and size of the dyed product according to the embodiment of the present disclosure are not particularly limited and may be appropriately selected as desired.

The material of the dyed product according to the embodiment of the present disclosure is not particularly limited, but is preferably hair.

### (Azo coloring agent)

The azo coloring agent according to the embodiment of the present disclosure is an azo coloring agent represented by Formula 1 or 2. The azo coloring agent according to the embodiment of the present disclosure has excellent dyeing properties and excellent washing resistance of a dyed product to be obtained.

The preferred aspects of the azo coloring agent represented by Formula 1 or 2 in the azo coloring agent according to the embodiment of the present disclosure are the same as the preferred aspects of the azo coloring agent represented by Formula 1 or 2 in the above-described dyeing composition except for the description below.

From the viewpoints of the dyeing properties and the washing resistance of a dyed product to be obtained, it is preferable that the azo coloring agent according to the embodiment of the present disclosure is an azo coloring agent represented by Formula 1-A as a compound represented by Formula 1.

A^{1A}-N=N-C^{1A}-N=N-B^{1A} Formula 1-A

In Formula 1-A, C^{1A} represents a group represented by any one of Formulae (C-1), (C-2), (C-3), (C-4), and (C-5), A^{1A} represents an aromatic group or a group represented by any one of Formulae (A-1), (A-9), (A-14), and (A-23), B^{1A} represents a group represented by any one of Formulae (B-5), (B-6), (B-7), (B-13), (B-14), and (B-15), and none of A^{1A}, B^{1A}, and C^{1A} has a carboxy group, a sulfo group, -SO₂F, or a cation structure.

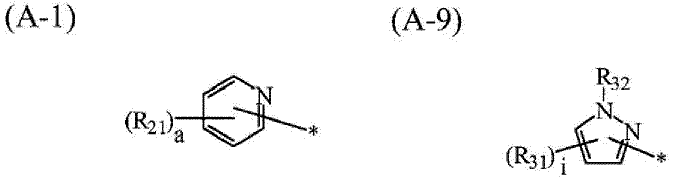

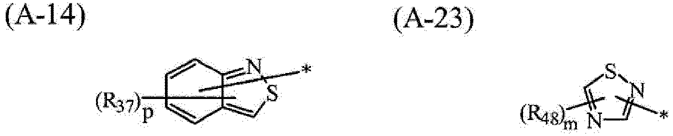

In Formulae (A-1), (A-9), (A-14), and (A-23),
* represents a position bonded to an azo group in Formula 1-A,
R₂₁, R₃₁, R₃₂, R₃₇, and R₄₈ each independently represent a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
adjacent groups among R₂₁, R₃₁, R₃₂, R₃₇, and R₄₈ may be bonded to each other to form a saturated or unsaturated 5- or 6-membered ring structure,
a and p each independently represent an integer of 0 to 4,
i represents an integer of 0 to 2, and
m represents 0 or 1.

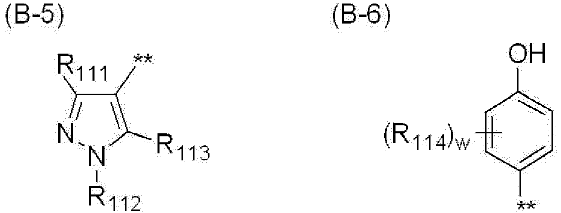

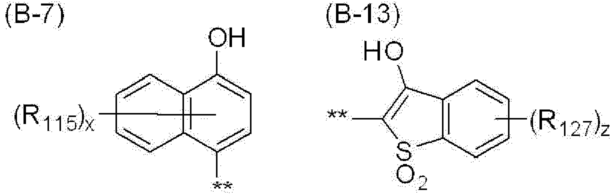

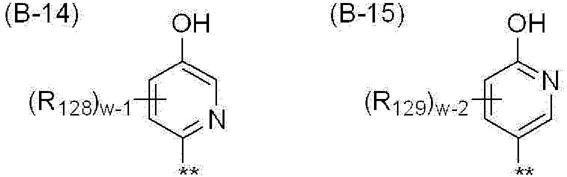

In Formulae (B-5), (B-6), (B-7), (B-13), (B-14), and (B-15),
** represents a position bonded to an azo group in Formula 1-A,
R₁₁₁ represents an alkyl group, an aryl group, an alkoxy group, an amino group (including an anilino group), an alkoxycarbonyl group, a cyano group, an acylamino group, or a carbamoyl group,
R₁₁₂ represents a hydrogen atom, an alkyl group, an aryl group, or a heterocyclic group,
R₁₁₃ represents a hydroxy group or an amino group,
R₁₁₄ and R₁₁₅ each independently represent a halogen atom, an alkyl group, an aryl group, a heterocyclic group, a nitro group, an alkoxy group, an aryloxy group, an amino group (including an anilino group), an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w represents an integer of 0 to 4,
x represents an integer of 0 to 6,
R₁₂₇'s each independently represent a halogen atom, an alkyl group, an aryl group, a heterocyclic group, a cyano group, a hydroxy group, a nitro group, an alkoxy group, an aryloxy group, an amino group (including an anilino group), an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
z represents an integer of 0 to 4,
R₁₂₈ and R₁₂₉ each independently represent a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w-1 represents an integer of 0 to 3, and
w-2 represents an integer of 0 to 3.

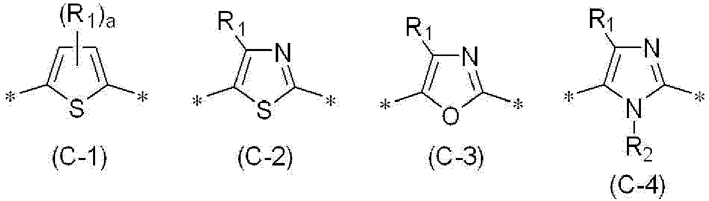

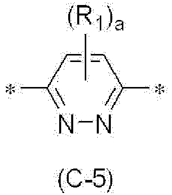

In Formulae (C-1), (C-2), (C-3), (C-4), and (C-5),
R₁'s each independently represent a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
a represents an integer of 0 to 2,
R₂ represents a hydrogen atom, an alkyl group, or an aryl group, and
* represents a position bonded to an azo group in Formula 1-A.

Further, from the viewpoints of the dyeing properties and the washing resistance of a dyed product to be obtained, it is preferable that the azo coloring agent according to the embodiment of the present disclosure is an azo coloring agent represented by Formula 2-A as a compound represented by Formula 2.

D^{1A}N=N-E^{1A} Formula 2-A

In Formula 2-A, D^{1A} represents an anthraquinonyl group, and E^{1A} represents a group represented by Formula E-1. 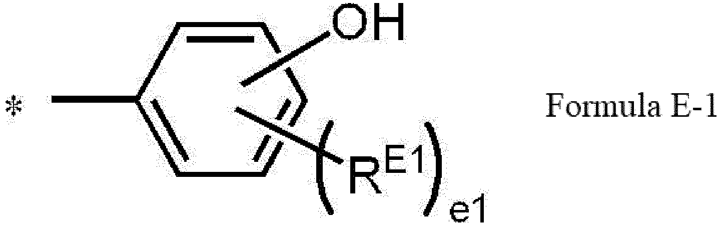 In Formula E-1, * represents a position bonded to an azo group in Formula 2-A, R^{E1}'s each independently represent a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, and e1 represents an integer of 1 to 4.

In a case where E^{1A} represents a group represented by Formula E-1, the dyeing properties and the washing resistance of a dyed product to be obtained are further excellent.

### Examples

Hereinafter, the present disclosure will be described in detail with reference to examples, but the present disclosure is not limited thereto. In the examples, "%" and "parts" respectively indicate "% by mass" and "parts by mass" unless otherwise specified.

Further, compounds 1 to 22 used in the examples are respectively the same as the compounds 1 to 22 described above.

### <Synthesis of compound 1>

5.0 g of a compound A (manufactured by Fujifilm Fine Chemicals Co., Ltd.) was added to 35.0 mL of 85% phosphoric acid (manufactured by Fujifilm Wako Pure Chemical Corporation), and the mixture was stirred at 25°C so as to be dissolved. Next, 1.96 g of sodium nitrite (manufactured by Fujifilm Wako Pure Chemical Corporation) was added thereto under ice cooling while the internal temperature was maintained at 0°C to 5°C, and the solution was stirred at the same temperature for 30 minutes.

30 mL of methanol was added to 2.93 g of a compound B (manufactured by Tokyo Chemical Industry Co., Ltd.) and dissolved. Next, a solution of the compound A in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated compound C was filtered and washed with acetonitrile. The obtained crystals were suspended in 100 mL of ion exchange water in an undried state, and potassium carbonate (manufactured by Kanto Chemical Co., Inc.) was added thereto until the pH of the water reached approximately 8. Thereafter, the solution was stirred for 60 minutes while the temperature thereof was maintained at 25°C to 30°C, and the precipitated crystals were filtered, thereby obtaining 4.8 g of a compound D as orange crystals.

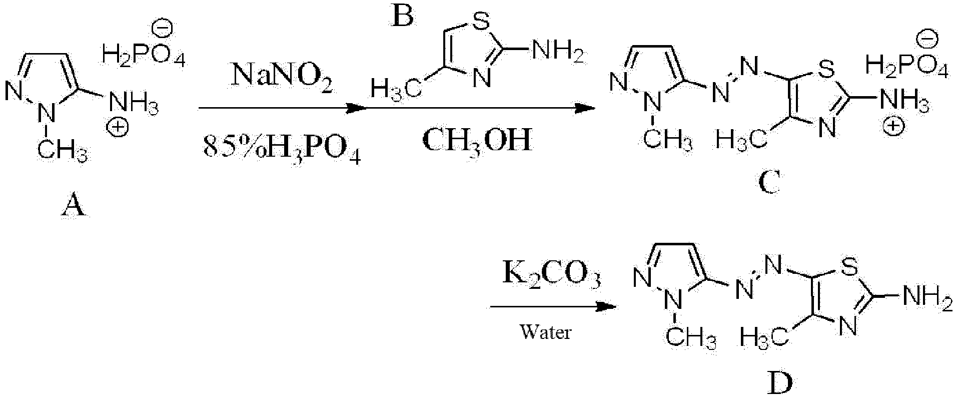

Next, 3.5 g of the compound D was added to 35.0 mL of 85% phosphoric acid (manufactured by Fujifilm Wako Pure Chemical Corporation), and the mixture was stirred at 25°C so as to be dissolved. Next, 1.20 g of sodium nitrite (manufactured by Fujifilm Wako Pure Chemical Corporation) was added thereto under ice cooling while the internal temperature was maintained at 0°C to 5°C, and the solution was stirred at the same temperature for 30 minutes, thereby obtaining a solution of the compound D in diazonium salt phosphoric acid.

20 mL of methanol was added to 2.02 g of a compound E (manufactured by Tokyo Chemical Industry Co., Ltd.) and dissolved. Next, a solution of the compound D in diazonium salt phosphoric acid was added dropwise thereto while the temperature was maintained at 0°C or lower during cooling with ice and methanol. After the addition, the resulting solution was stirred at 0°C to 5°C for 30 minutes, 100 mL of ion exchange water was added thereto, and the precipitated crystals were filtered. The obtained crystals were dried and then purified by silica gel chromatography, thereby obtaining 4.0 g of a compound 1 as brown purple crystals.

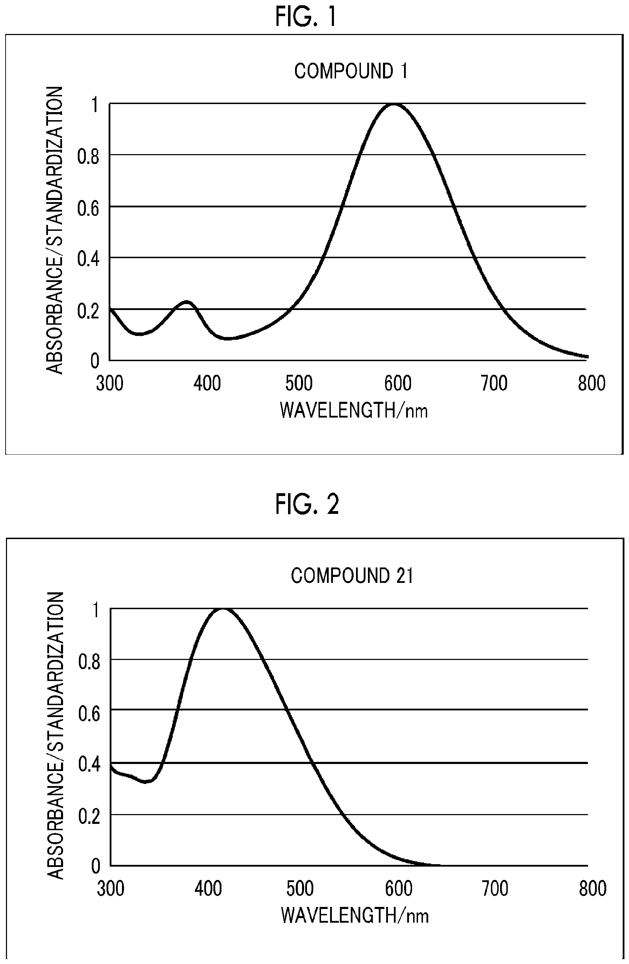

As a result of confirmation of the structure of the compound obtained in the above-described manner based on NMR, ¹H-NMR (300 MHz, solvent: dimethylsulfoxide-d₆, standard substance: tetramethylsilane) δ = 8.9 ppm (1H, s), 8.8 ppm (1H, d), 7.6 ppm (1H, d), 7.1 ppm (1H, d), 4.1 ppm (3H, s), 2.8 ppm (3H, s). λmax (2-aminoethanol solution) of the absorption spectrum was 598 nm, and ε (the same) was 69000. The absorption chart is shown in Fig. 1.

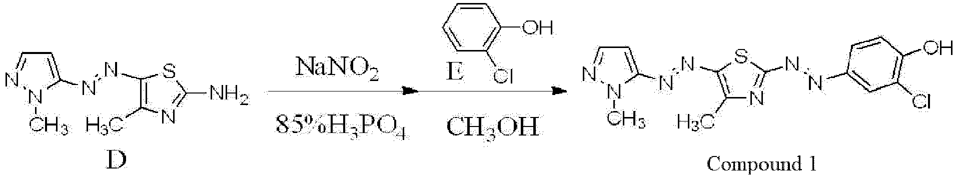

### <Synthesis of compound 13>

10.0 g of 2-acetylthiophene was dissolved in 71 g of ethyl acetate, 5.0 g of tetrabutylammonium bromide was added thereto, and the solution was heated until the internal temperature reached 37°C to 43°C. After the internal temperature was maintained at 37°C to 43°C and 15 g of bromine was added dropwise to the reaction system, the solution was stirred at the same temperature for 1 hour. Next, 6 g of thiourea and 60 g of water were added thereto. After the resulting solution was stirred at an internal temperature of 37°C to 43°C for 1 hour, the solution was air-cooled and stirred until the internal temperature reached 30°C or lower. After the solid in the reaction solution was collected by filtration, the obtained solid was washed with ethyl acetate and dried, thereby obtaining 16.2 g of a compound F as a brown solid.

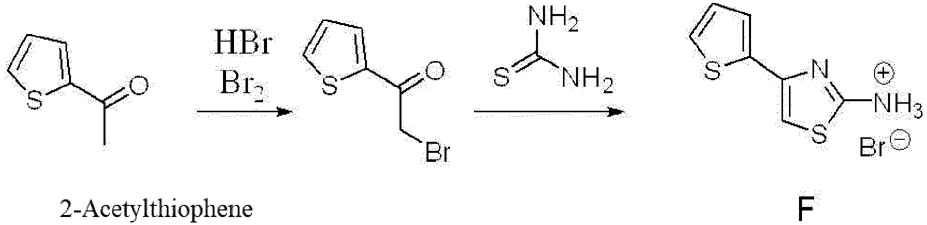

Next, 5.0 g of the compound A was added to 35.0 mL of 85% phosphoric acid, and the mixture was stirred at 25°C so as to be dissolved. Next, 1.96 g of sodium nitrite was added thereto under ice cooling while the internal temperature was maintained at 0°C to 5°C, and the solution was stirred at the same temperature for 30 minutes.

30 mL of methanol was added to 6.74 g of the compound F and dissolved. Next, a solution of the compound A in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated solid was filtered and washed with acetonitrile. The obtained solid was dried, thereby obtaining 8.3 g of a compound G as orange crystals.

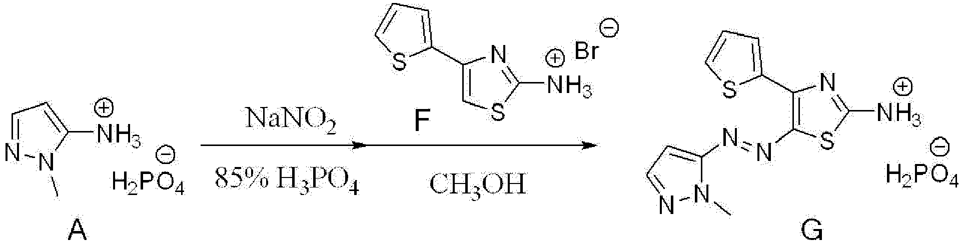

Next, 3.2 g of the compound G was added to 32.0 mL of 85% phosphoric acid, and the mixture was stirred at 25°C so as to be dissolved. Next, 0.63 g of sodium nitrite was added thereto under ice cooling while the internal temperature was maintained at 0°C to 5°C, and the solution was stirred at the same temperature for 30 minutes, thereby obtaining a solution of the compound G in diazonium salt phosphoric acid.

30 mL of methanol was added to 1.1 g of the compound E and dissolved. Next, a solution of the compound G in diazonium salt phosphoric acid was added dropwise thereto while the temperature was maintained at -5°C or lower during cooling with ice and methanol. After the addition, the resulting solution was stirred at -5°C to 0°C for 1 hour, 45 mL of acetonitrile was added thereto, and a precipitated solid was filtered and washed with acetonitrile. The obtained solid was dried and then purified by silica gel chromatography, thereby obtaining 2.5 g of a compound 13 as a dark brown solid.

As a result of confirmation of the structure of the compound 13 obtained in the above-described manner based on NMR, ¹H-NMR (400 MHz, solvent: dimethylsulfoxide-d₆, standard substance: tetramethylsilane) δ = 8.09 ppm (1H, s), 8.05 (1H, s), 7.94-7.91 ppm (2H, m), 7.7 ppm (1H, s), 7.3 ppm (1H, s), 7.2 ppm (1H, d), 6. 7 ppm (1H, s), 4.1 ppm (3H, s). λmax (dimethylformamide) of the absorption spectrum was 682 nm, and ε (the same) was 54300.

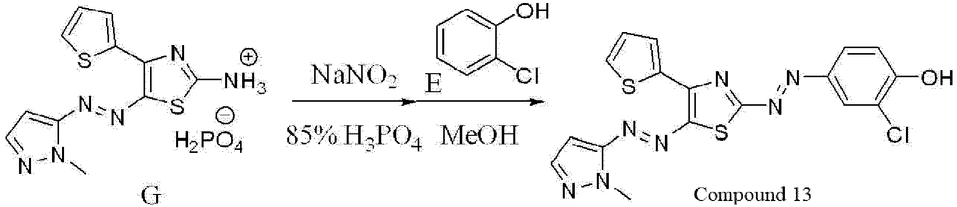

### <Synthesis of compound 12>

After 20.0 g of 3-acetylpyridine was added dropwise to 27.5 mL of a 47% aqueous solution of hydrogen bromide while the internal temperature was maintained at 30°C or lower, the solution was heated and stirred until the internal temperature reached 57°C to 63°C. Next, 26.4 g of bromine was added dropwise thereto, the solution was stirred at an internal temperature of 67 to 73°C for 1 hour and air-cooled until the internal temperature reached 30°C or lower. The precipitated solid was collected by filtration, and 165 g of water and 12.6 g of thiourea were added thereto. The resulting solution was stirred at an internal temperature of 20°C to 25°C for 1 hour and neutralized such that the pH thereof reached 6 to 7 with a saturated aqueous solution of sodium hydrogen carbonate. After the solid in the reaction solution was collected by filtration, the obtained solid was washed with water and dried, thereby obtaining 30.0 g of a compound H as a pale yellow solid.

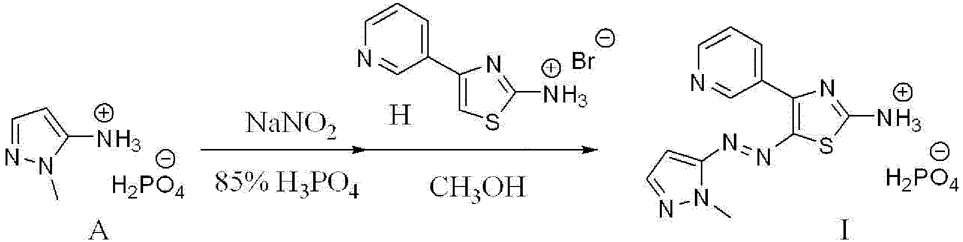

Next, 5.0 g of the compound A was added to 35.0 mL of 85% phosphoric acid, and the mixture was stirred at 25°C so as to be dissolved. Next, 1.96 g of sodium nitrite was added thereto under ice cooling while the internal temperature was maintained at 0°C to 5°C, and the solution was stirred at the same temperature for 30 minutes.

30 mL of methanol was added to 4.54 g of the compound H and dissolved. Next, a solution of the compound A in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated solid was filtered and washed with acetonitrile. The obtained solid was dried, thereby obtaining 7.2 g of a compound I as orange crystals.

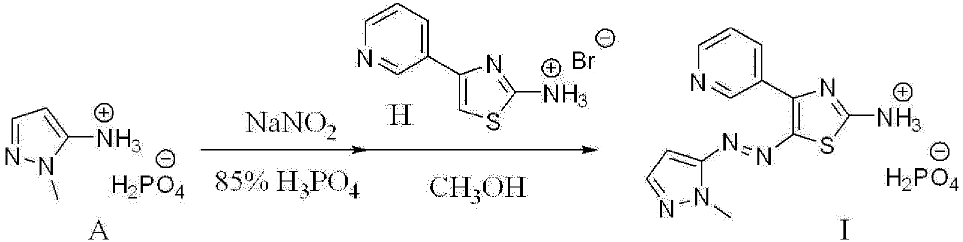

Next, 4.0 g of the compound I was added to 35.0 mL of 85% phosphoric acid, and the mixture was stirred at 25°C so as to be dissolved. Next, 0.79 g of sodium nitrite was added thereto under ice cooling while the internal temperature was maintained at 0°C to 5°C, and the solution was stirred at the same temperature for 30 minutes.

30 mL of methanol was added to 2.0 g of the compound E and dissolved. Next, a solution of the compound I in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated solid was filtered and washed with acetonitrile. The obtained solid was dried and then purified by silica gel chromatography, thereby obtaining 3.1 g of a compound 12 as orange crystals.

As a result of confirmation of the structure of the compound 12 obtained in the above-described manner based on NMR, ¹H-NMR (400 MHz, solvent: dimethylsulfoxide-d₆, standard substance: tetramethylsilane) δ = 9.4 ppm (1H, s), 8.7 (1H, d), 8.6 ppm (1H, d), 8.0 ppm (1H, d), 7.7-7.6 ppm (2H, m), 7.2 ppm (1H, s), 6.7 ppm (1H, s), 6.6 ppm (1H, s), 4.1 ppm (3H, s). λmax (dimethylformamide) of the absorption spectrum was 682 nm, and ε (the same) was 52600.

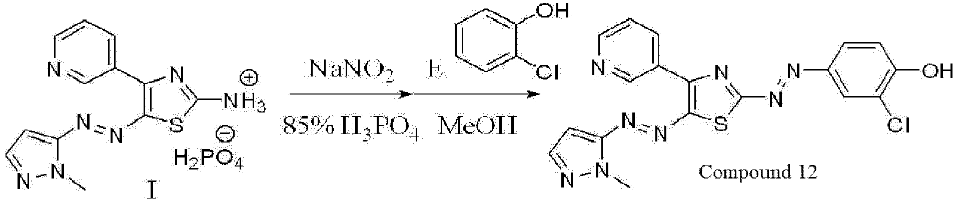

### <Synthesis of compound 11>

9 g of thiourea was suspended in 55 g of ethanol and dissolved by being heated, and the solution was stirred under reflux. 24 g of phenacyl bromide was added thereto, and the resulting solution was stirred under reflux for 3 hours. The reaction solution was air-cooled and stirred until the internal temperature thereof reached 27°C to 33°C, and the precipitated solid was collected by filtration. The obtained solid was dried, thereby obtaining 25 g of a compound J as a pale yellow solid.

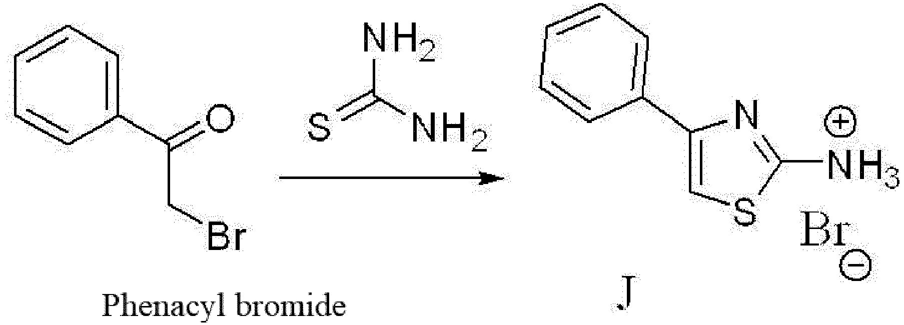

Next, 5.0 g of the compound A was added to 35.0 mL of 85% phosphoric acid, and the mixture was stirred at 25°C so as to be dissolved. Next, 1.96 g of sodium nitrite was added thereto under ice cooling while the internal temperature was maintained at 0°C to 5°C, and the solution was stirred at the same temperature for 30 minutes, thereby obtaining a solution of the compound A in diazonium salt phosphoric acid.

30 mL of methanol was added to 6.59 g of the compound J and dissolved. Next, a solution of the compound A in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated solid was filtered and washed with acetonitrile. The obtained solid was dried, thereby obtaining 6.5 g of a compound K as orange crystals.

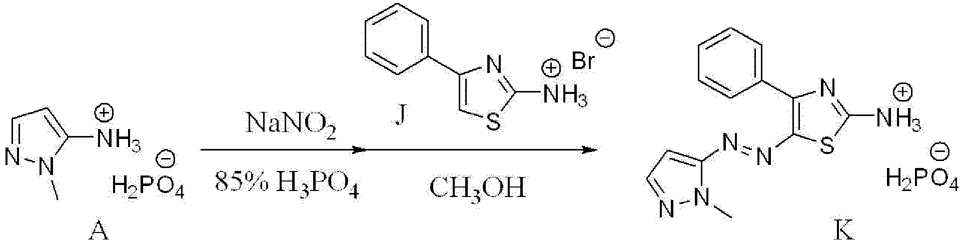

Next, 4.0 g of the compound K was added to 35.0 mL of 85% phosphoric acid, and the mixture was stirred at 25°C so as to be dissolved. Next, 0.79 g of sodium nitrite was added thereto under ice cooling while the internal temperature was maintained at 0°C to 5°C, and the solution was stirred at the same temperature for 30 minutes, thereby obtaining a solution of the compound K in diazonium salt phosphoric acid.

30 mL of methanol was added to 1.4 g of the compound E and dissolved. Next, a solution of the compound K in diazonium salt phosphoric acid was added dropwise thereto while the temperature was maintained at 0°C or lower during cooling with ice and methanol. After the addition, the resulting solution was stirred at 0°C to 5°C for 30 minutes, 45 mL of acetonitrile was added thereto, and a precipitated solid was filtered and washed with acetonitrile. The obtained solid was dried and then purified by silica gel chromatography, thereby obtaining 2.2 g of a compound 11 as orange crystals.

As a result of confirmation of the structure of the compound 11 obtained in the above-described manner based on NMR, ¹H-NMR (400 MHz, solvent: dimethylsulfoxide-d₆, standard substance: tetramethylsilane) δ = 8.29 ppm (1H, s), 8.28 (1H, s), 8.0 ppm (1H, s), 7.9 ppm (1H, d), 7.7 ppm (1H, s), 7.6-7.5 ppm (3H, m), 7.2 ppm (1H, d), 6.7 ppm (1H, s), 4.1 ppm (3H, s). λmax (dimethylformamide) of the absorption spectrum was 679 nm, and ε (the same) was 50200.

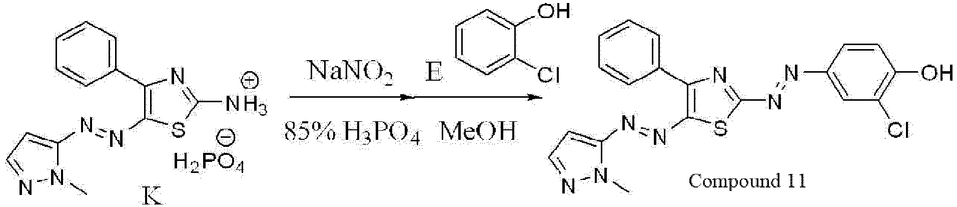

### <Synthesis of compound 21>

A mixture of 3.3 g of 1-aminoanthraquinone, 5.6 g of concentrated hydrochloric acid, 3.9 g of ion exchange water, and 17.0 g of acetic acid was stirred and cooled in an ice water bath. A solution of 1.2 g of sodium nitrite and 1.8 g of ion exchange water was added dropwise thereto, the mixture was further stirred under ice cooling for 30 minutes, and 4.4 g of sodium acetate was added to the reaction mixture. A solution of 2.3 g of 2-chlorophenol, 0.6 g of sodium hydroxide, and 45 mL of methanol was stirred and cooled in an ice water bath, and the above-described reaction mixture was added dropwise thereto. The solution was stirred at room temperature (25°C, the same applies hereinafter) for 2 hours, and the precipitated crystals were collected by filtration and washed with methanol. The obtained crystals were dried and dissolved in 45 mL of N,N-dimethylacetamide, and 45 mL of methanol and 23 mL of ion exchange water were sequentially added thereto. The precipitated crystals were collected by filtration, washed with methanol, and dried, thereby obtaining 4.1 g of a compound 21.

As a result of confirmation of the structure of the compound 21 obtained in the above-described manner based on NMR, ¹H-NMR (400 MHz, dimethylsulfoxide (DMSO)-d₆) δ = 7.1 (d, 1H), 7.3 (d, 1H), 7.8-8.0 (m, 6H), 8.2 (d, 1H), 8.3 (s, 1H), 11.2 (s, 1H).

### <Synthesis of compound 22>

A mixture of 3.3 g of 2-aminoanthraquinone and 30 mL of 85% phosphoric acid was stirred and cooled in an ice water bath. 1.2 g of sodium nitrite was divided and added thereto, and the mixture was further stirred for 2 hours under ice cooling. A solution of 2.3 g of 2-chlorophenol, 3.8 g of a 28% sodium methoxide methanol solution, and 30 mL of methanol was stirred and cooled in an ice water bath, and the above-described reaction mixture was added dropwise thereto. The solution was stirred at room temperature for 2 hours, and 69.5 g of a 28% sodium methoxide methanol solution was added dropwise thereto, and the resulting solution was further stirred for 2 hours. The precipitated crystals were collected by filtration and washed with ion exchange water. The obtained crystals were dried, 120 mL of tetrahydrofuran was added thereto, and insolubles were removed by filtration. The filtrate was concentrated under reduced pressure until the amount thereof reached 20 mL, and 20 mL of ethyl acetate was added thereto. The precipitated crystals were collected by filtration, washed with ethyl acetate, and dried, thereby obtaining 3.2 g of a compound 22.

As a result of confirmation of the structure of the compound 22 obtained in the above-described manner based on NMR, ¹H-NMR (400 MHz, DMSO-d₆) δ = 7.1 (d, 1H), 7.8-8.0 (m, 5H), 8.1 (d, 1H), 8.2 (d, 1H), 8.3 (d, 1H), 11.3 (s, 1H).

### (Examples 1 to 3 and Comparative Examples 1 and 2)

### <Measurement of solution absorption characteristics>

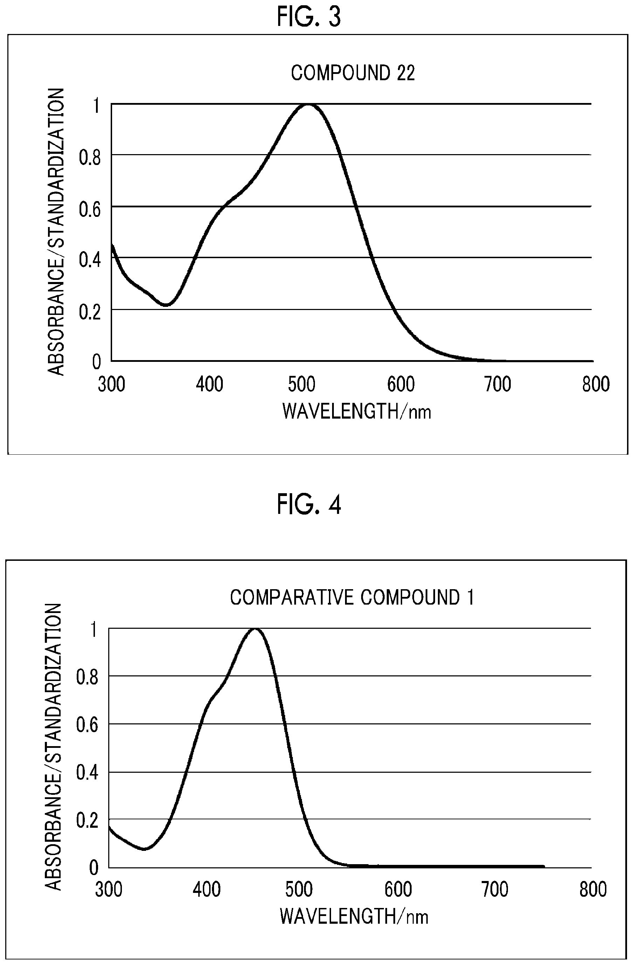

A solution of each dye (compound 1: Example 1, compound 21: Example 2, compound 22: Example 3, comparative compound 1: Comparative Example 1, comparative compound 2: Comparative Example 2) with a concentration of 1 × 10⁻⁵ to 1 × 10⁻⁴ mol/L was prepared, and the absorption spectrum was measured using an ultraviolet-visible spectrophotometer UV-1800 (manufactured by Shimadzu Corporation). The measurement results are listed in Table 2. Further, the absorption spectra (transmission spectra) of the compound 1, the compound 21, the compound 22, the comparative compound 1, and the comparative compound 2 are respectively shown in Figs. 1 to 5.

As an index indicating that the compound had an absorption in a wide region, the absorption width imparting an absorption intensity of 0.4 at the time of setting the absorption intensity at the maximum absorption to 1 was shown in the unit of nm.

In addition, a numerical value with a large absorption width indicates that the compound has an absorption in a wider region, and dyeing of a color with low brightness and saturation which is not vivid can be carried out, and the color of the dyed product is a dull color tone.

**[Table 2]**

| Dye | Solvent | Absorption width (nm) | Maximum absorption wavelength(nm) |
|---|---|---|---|
| Compound 1 | 5 mass% ethanolamine aqueous solution | 160 | 598 |
| Compound 21 | 5 mass% ethanolamine aqueous solution | 155 | 418 |
| Compound 22 | 5 mass% ethanolamine aqueous solution | 185 | 505 |
| Comparative compound 1 | 5 mass% ethanolamine aqueous solution | 110 | 450 |
| Comparative compound 2 | 5 mass% ethanolamine aqueous solution | 165 | 643 |

In addition, the comparative compounds 1 and 2 are the compounds shown below.

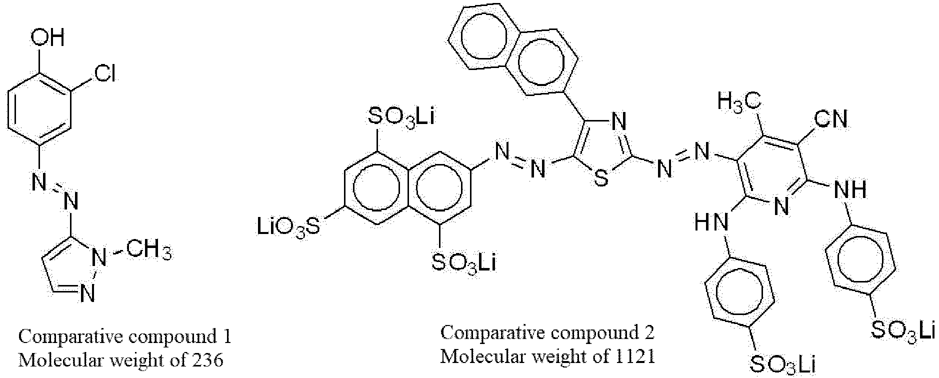

The compounds 1, 21, and 22 had an absorption in a wider region than that in Comparative Compound 1, and thus a tint that was not vivid was exhibited.

### <Dyeing test and washing resistance test>

### - Dyeing test -

A mixed solution of 11.5 mg of each dye, 8.6 g of the dyeing solvent listed in Table 3, and 1 ml of a 28% ammonia aqueous solution was prepared, and silk (5 cm × 5 cm) and white goat hair (approximately 1 g) were immersed in the mixed solution at 40°C for 2 hours.

The immersed material was sufficiently rinsed with running water and then dried. The reflection spectrum of the dried sample was measured using UV-3100PC (ultraviolet-visible infrared spectrophotometer) (manufactured by Shimadzu Corporation), and the transmittance at a maximum absorption wavelength of each dye was used as an index of the dyeing properties.

A lower transmittance indicates that the dyeing properties are further excellent, and a transmittance of 60% or greater is at a level where the sample was slightly colored.

Further, the results of visually evaluating the tint of the dyed product, and measuring the Lab color space with an X-rite (manufactured by X-Rite Inc.) under measurement conditions of a light source status T and a viewing angle of 2 degrees are listed in Table 3.

The Lab color space indicates a CIE1976L*a*b* color space. Further, the details of the CIE1976L*a*b* color space are described in JIS standard "JIS Z 8781-4 (Part 4: CIE1976L*a*b* color space)".

### - Washing resistance test -

The dyed sample was immersed in 100 ml of ion exchange water at 25°C for 1 hour. The liquid after immersion was placed in a quartz cell having an optical path length of 1 cm, and the absorption spectrum was measured with an ultraviolet-visible spectrophotometer UV-1800 (manufactured by Shimadzu Corporation).

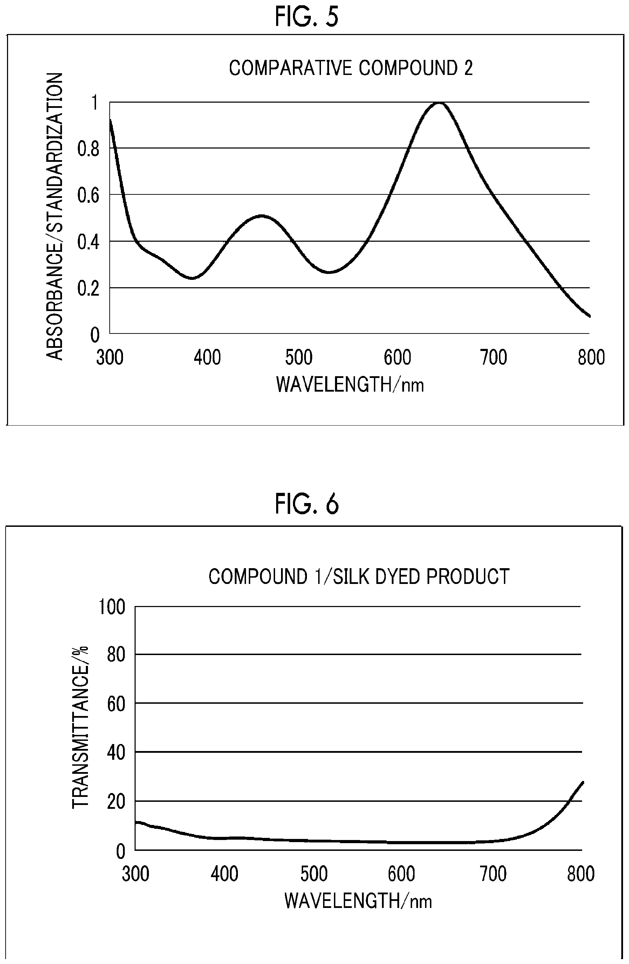

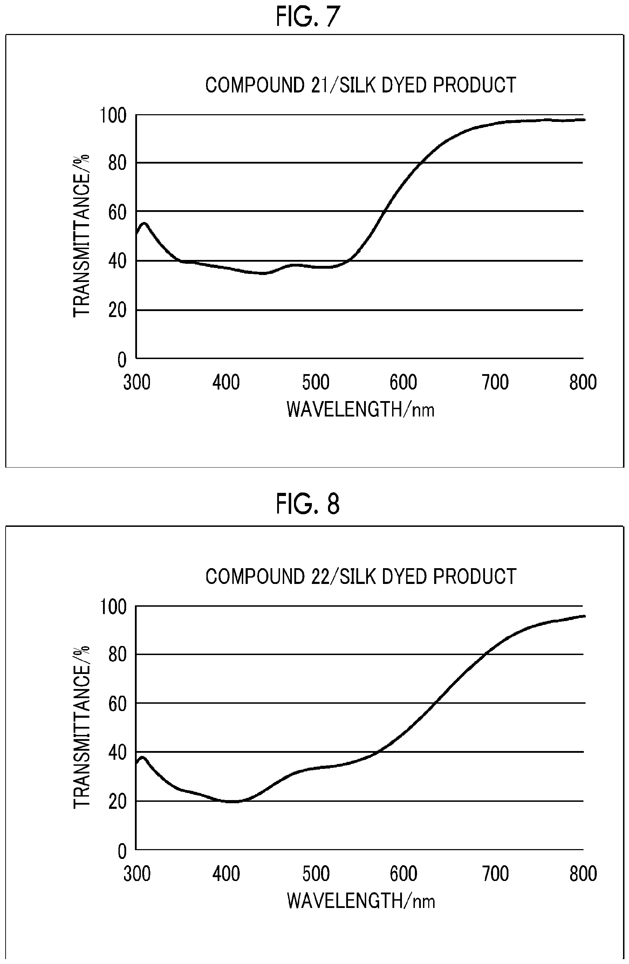

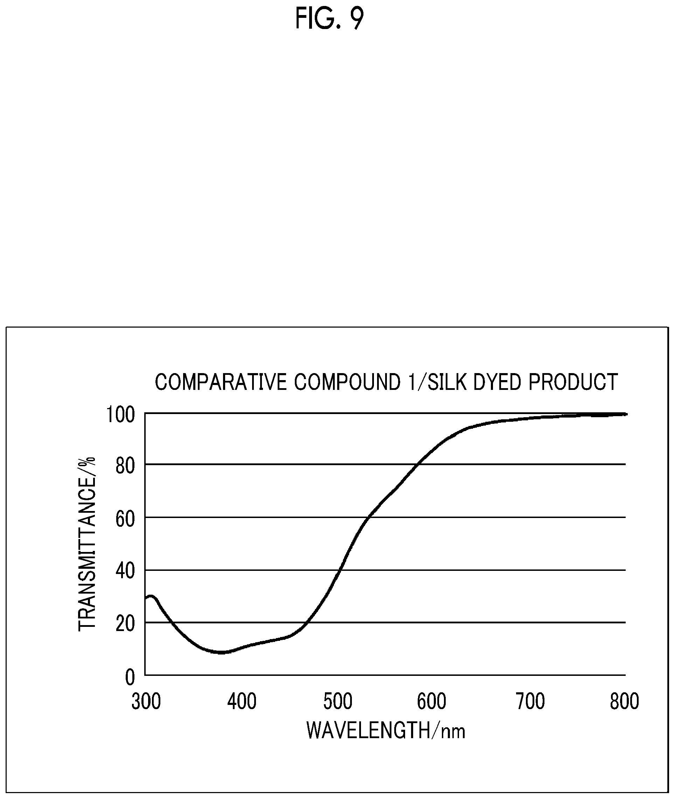

The absorbance at the maximum absorption wavelength of each dye is listed in Table 4. The reflection spectra in a case of using the compounds 1, 21, and 22 and Comparative Compound 1 are respectively shown in Figs. 6 to 9.

As the value of the absorbance increases, this indicates that the dye was eluted from the dyed sample, and the washing resistance was degraded.

**[Table 3]**

| | Dyeing solvent | Tint of dyed product | Dyeing properties | | | | | | | |
|---|---|---|---|---|---|---|---|---|---|---|
| | | | Silk | | | | Goat hair | | | |
| | | | Transmittance % | L* | a* | b* | Transmittance % | L* | a* | b* |
| Compound 1 | Ion exchange water | Bluish gray | 4 | 20.7 | -0.5 | -3.0 | 24 | 21.9 | -4.5 | -23.4 |
| Compound 21 | Methanol | Reddish gray | 35 | 75.0 | 24.4 | 20.2 | 31 | 69.7 | 6.6 | 17.7 |
| Compound 22 | Methanol | Brown | 34 | 66.3 | 12.2 | 19.7 | 34 | 60.8 | 11.8 | 13.5 |
| Comparative compound 1 | Ion exchange water | Vivid yellow | 9 | 82.8 | 8.4 | 56.3 | 16 | 64.2 | 8.5 | 67.0 |
| Comparative compound 2 | Ion exchange water | * 1 | 80 | 86.7 | -2.0 | 0.7 | 65 | 75.6 | 0.2 | 11.4 |

| | | | | | | | | | | |
|---|---|---|---|---|---|---|---|---|---|---|
| *1 Since the dyed product was slightly colored, the tint was not able to be determined. | | | | | | | | | | |

As listed in Table 3, since the compounds 1, 21, and 22 exhibit excellent dyeing properties with respect to silk and goat hair and the compounds have an absorption in a wide region, a tint that is not vivid is shown.

**[Table 4]**

| | Dyeing solvent | Washing resistance | |
|---|---|---|---|
| | | Absorbance | |
| | | Silk | Goat hair |
| Compound 1 | Ion exchange water | 1.1 | 0.1 |
| Compound 2 | Methanol | 0.0 | 0.0 |
| Compound 22 | Methanol | 0.0 | 0.0 |
| Comparative compound 1 | Ion exchange water | 1.6 | 2.2 |
| Comparative compound 2 | Ion exchange water | *2 | *2 |

| | | | |
|---|---|---|---|
| *2 Since the dyed product was less colored, the evaluation was not able to be performed. | | | |

As listed in Table 4, in the compounds 1, 21 and 22, the result that the elution of the dye into the washing solution was small was obtained.

As described above, it was found that since the compounds 1, 21, and 22 had an absorption in a wide region, a color that was not vivid was exhibited, and excellent dyeing properties and excellent washing resistance were exhibited.

### (Example 4)

A dyeing test using white goat hair was performed in the same manner as in Example 1 except that the compound 1 in Example 1 was replaced with the compound 13.

As the result, neutral gray that was not vivid was exhibited.

### (Example 5)

A dyeing test using white goat hair was performed in the same manner as in Example 1 except that the compound 1 in Example 1 was replaced with the compound 12.

As the result, bluish gray that was not vivid was exhibited.

### (Example 6)

A dyeing test using white goat hair was performed in the same manner as in Example 1 except that the compound 1 in Example 1 was replaced with the compound 11.

As the result, bluish gray that was not vivid was exhibited.

The disclosure of JP2018-014808 filed on January 31, 2018 is incorporated herein by reference in its entirety.

All documents, patent applications, and technical standards described in the present specification are incorporated herein by reference to the same extent as in a case of being specifically and individually noted that individual documents, patent applications, and technical standards are incorporated by reference.

## Claims

1. A dyeing composition comprising:
an azo coloring agent represented by Formula 1 or 2,
A¹-N=N-C¹-N=N-B¹ Formula 1
D¹-N=N-E¹ Formula 2
wherein, in Formulae 1 and 2,
C¹ represents a heteroaromatic group,
each of A¹ and B¹ independently represents a heteroaromatic group, an aromatic group, or a group having a methine structure, and at least one of A¹ or B¹ represents a heteroaromatic group,
none of A¹, B¹, and C¹ has a carboxy group, a sulfo group, -SO₂F, or a cation structure,
D¹ represents an anthraquinonyl group,
E¹ represents a heteroaromatic group, an aromatic group, or a group having a methine structure, the heteroaromatic group as E¹ is a heteroaromatic group which has been substituted with a hydroxy group or a monosubstituted or unsubstituted amino group on a heteroaromatic ring or a heteroaromatic group which has an NH moiety in a ring structure, and the aromatic group as E¹ is an aromatic group which has been substituted with a hydroxy group or a monosubstituted or unsubstituted amino group on an aromatic ring, and
neither D¹ nor E¹ has a carboxy group or a sulfo group.

2. The dyeing composition according to claim 1,
wherein C¹ in Formula 1 represents a group represented by any one of Formulae (C-1) to (C-7), 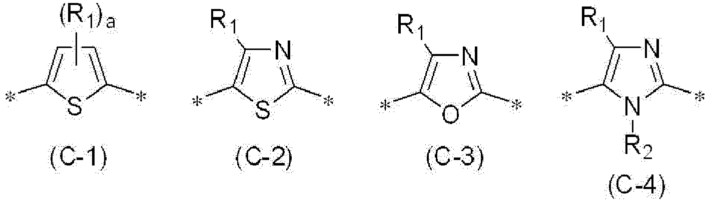 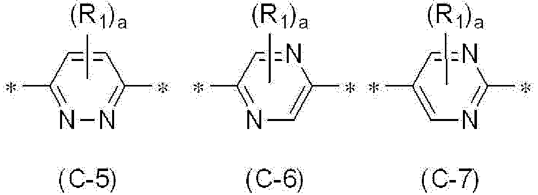 wherein, in Formulae (C-1) to (C-7),
each R₁ independently represents a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
a represents an integer from 0 to 2,
R₂ represents a hydrogen atom, an alkyl group, or an aryl group, and
* represents a position bonded to an azo group in Formula 1.

3. The dyeing composition according to claim 1 or 2,
wherein A¹ in Formula 1 represents an aromatic group or a group represented by any one of Formulae (A-1) to (A-25), and
B¹ represents a group represented by any one of Formulae (B-1) to (B-15), 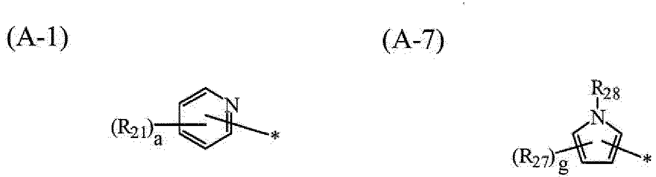 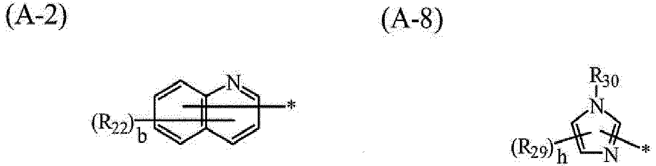 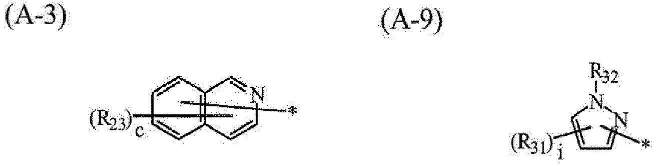 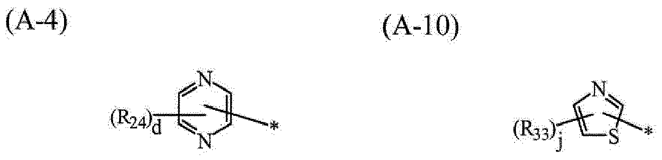 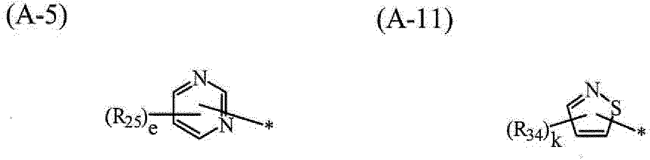 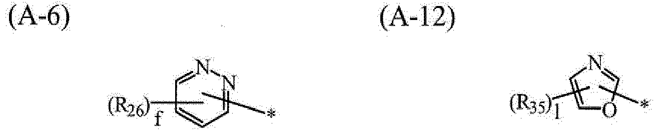 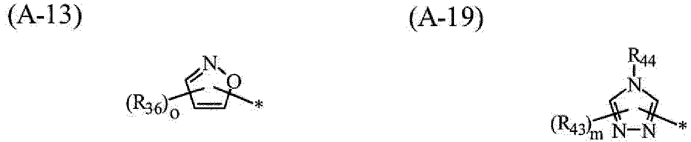 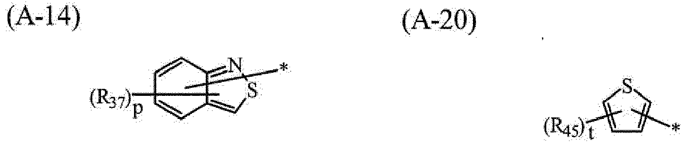 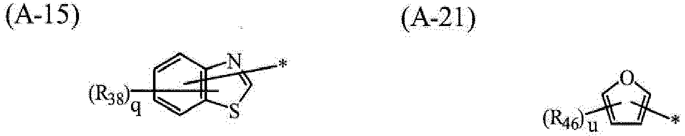 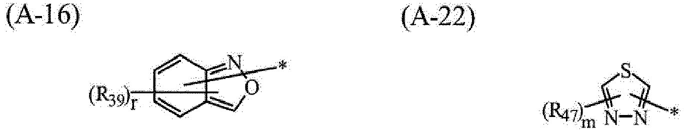 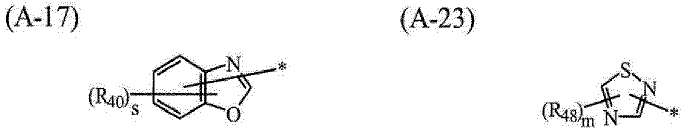 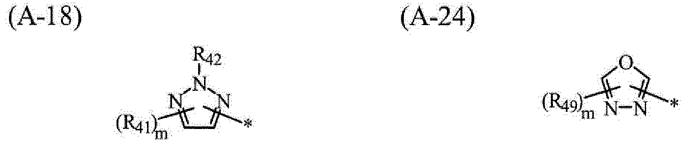 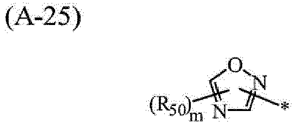
wherein, in Formulae (A-1) to (A-25),
* represents a position bonded to an azo group in Formula 1,
each of R₂₁ to R₅₀ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
adjacent groups among R₂₁ to R₅₀ may be bonded to each other to form a saturated or unsaturated 5- or 6-membered ring structure,
each of a, p, q, r, and s independently represents an integer from 0 to 4,
each of b and c independently represents an integer from 0 to 6,
each of d, e, f, g, t, and u independently represents an integer from 0 to 3,
each of h, i, j, k, l, and o independently represents an integer from 0 to 2, and
m represents 0 or 1; 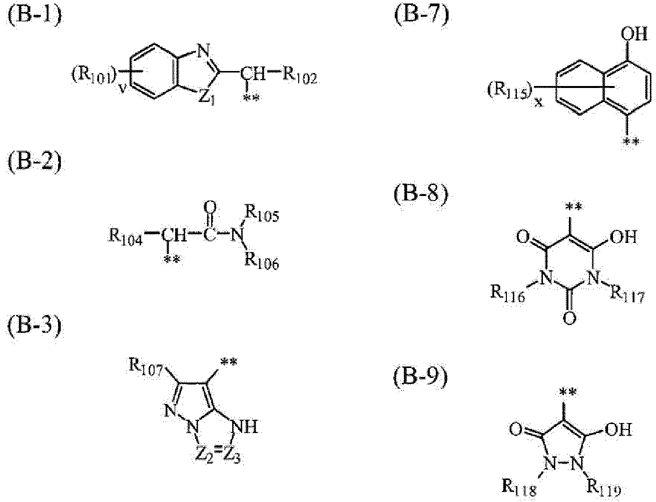 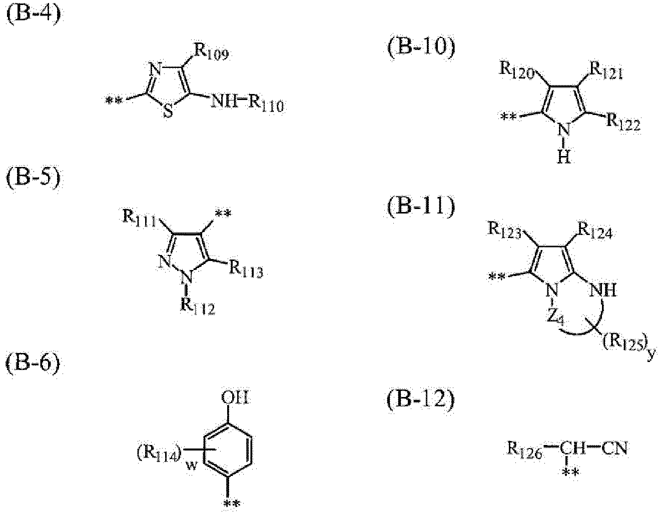 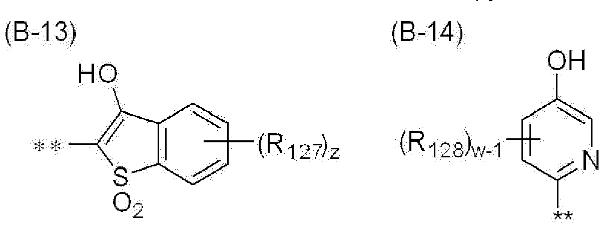 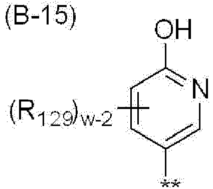
wherein, in Formulae (B-1) to (B-15),
** represents a position bonded to an azo group in Formula 1,
each R₁₀₁ independently represents a halogen atom, an alkyl group, an aryl group, a heterocyclic group, a cyano group, an alkoxy group, an amino group, an acylamino group, an alkylsulfonylamino group, an arylsulfonylamino group, an alkylthio group, an arylthio group, a sulfamoyl group, an alkylsulfonyl group, or a carbamoyl group,
v represents an integer from 0 to 4,
each of R₁₀₂ and R₁₀₄ independently represents a cyano group, an alkylsulfonyl group, an arylsulfonyl group, an acyl group, an alkoxycarbonyl group, an aryloxycarbonyl group, or a carbamoyl group,
Z₁ represents an oxygen atom, a sulfur atom, or -N(R₁₀₃)-,
R₁₀₃ represents a hydrogen atom, an alkyl group, an aryl group, or a heterocyclic group,
each of R₁₀₅ and R₁₀₆ independently represents a hydrogen atom, an alkyl group, an aryl group, or a heterocyclic group,
R₁₀₇ represents a hydrogen atom, an alkyl group, an aryl group, a heterocyclic group, an alkoxy group, an aryloxy group, an amino group (including an anilino group), an acylamino group, an alkylsulfonylamino group, an arylsulfonylamino group, an alkylthio group, an arylthio group, an alkylsulfonyl group, an arylsulfonyl group, or a carbamoyl group,
each of Z₂ and Z₃ independently represents -C(R₁₀₈)= or -N=,
each R₁₀₈ independently represents an alkyl group, an aryl group, a heterocyclic group, an alkylthio group, an arylthio group, an alkoxycarbonyl group, or a carbamoyl group,
two R₁₀₈'s may be bonded to each other to form a carbocyclic or heterocyclic ring in a case in which both Z₂ and Z₃ represent -C(R₁₀₈)=,
R₁₀₉ represents an alkyl group, an aryl group, or a heterocyclic group,
R₁₁₀ represents a hydrogen atom, an alkyl group, an aryl group, a heterocyclic group, an acyl group, an alkylsulfonyl group, or an arylsulfonyl group,
R₁₁₁ represents an alkyl group, an aryl group, an alkoxy group, an amino group, an alkoxycarbonyl group, a cyano group, an acylamino group, or a carbamoyl group,
R₁₁₂ represents a hydrogen atom, an alkyl group, an aryl group, or a heterocyclic group,
R₁₁₃ represents a hydroxy group or an amino group,
each of R₁₁₄ and R₁₁₅ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w represents an integer from 0 to 4,
x represents an integer from 0 to 6,
each of R₁₁₆, R₁₁₇, R₁₁₈, and R₁₁₉ independently represents an alkyl group or an aryl group,
each of R₁₂₀ and R₁₂₁ independently represents an alkyl group, an aryl group, a heterocyclic group, a cyano group, an alkylsulfonyl group, an arylsulfonyl group, an alkoxycarbonyl group, or a carbamoyl group,
R₁₂₂ represents a hydrogen atom, an alkyl group, an aryl group, a heterocyclic group, an acylamino group, an alkylsulfonylamino group, or an arylsulfonylamino group,
each of R₁₂₃ and R₁₂₄ independently represents an alkyl group, an aryl group, a heterocyclic group, a cyano group, an alkylsulfonyl group, an arylsulfonyl group, an alkoxycarbonyl group, or a carbamoyl group,
Z₄ represents a non-metallic atomic group that forms a 5- or 6-membered ring together with two nitrogen atoms and one carbon atom,
R₁₂₅ represents an alkyl group, an aryl group, an alkoxy group, an amino group, an acylamino group, an alkylsulfonylamino group, an arylsulfonylamino group, an alkylthio group, an arylthio group, an acyl group, an alkoxycarbonyl group, or a carbamoyl group,
y represents an integer from 0 to 2 in a case in which Z₄ forms a 5-membered ring,
y represents an integer from 0 to 3 in a case in which Z₄ forms a 6-membered ring,
R₁₂₆ represents an alkyl group or an aryl group,
each R₁₂₇ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
z represents an integer from 0 to 4,
each of R₁₂₈ and R₁₂₉ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w-1 represents an integer from 0 to 3, and
w-2 represents an integer from 0 to 3.

4. The dyeing composition according to any one of claims 1 to 3,
wherein the azo coloring agent represented by Formula 1 or 2 has a molecular weight of 600 or less.

5. The dyeing composition according to any one of claims 1 to 4,
wherein the azo coloring agent represented by Formula 1 or 2 has a molecular weight of 495 or less.

6. The dyeing composition according to claim 3, wherein:
A¹ in Formula 1 represents an aromatic group or a group represented by any one of Formulae (A-1), (A-9), (A-14) and (A-23), and
B¹ represents a group represented by any one of Formulae (B-1), (B-5), (B-6), (B-7), (B-9), (B-13), (B-14) and (B-15).

7. The dyeing composition according to claim 2,
wherein C¹ in Formula 1 represents a group represented by any one of Formulae (C-1) to (C-5).

8. The dyeing composition according to any one of claims 1 to 7,
wherein the azo coloring agent represented by Formula 1 or 2 contains at least one group selected from the group consisting of a hydroxy group, an amino group, and a cyano group.

9. The dyeing composition according to any one of claims 1 to 8,
wherein the azo coloring agent represented by Formula 1 or 2 contains a hydroxy group.

10. The dyeing composition according to any one of claims 1 to 9, comprising:
the azo coloring agent represented by Formula 1.

11. The dyeing composition according to any one of claims 1 to 9, comprising:
the azo coloring agent represented by Formula 2.

12. The dyeing composition according to any one of claims 1 to 11,
wherein the dyeing composition is a hair dyeing composition.

13. The dyeing composition according to claim 12,
wherein the dyeing composition is a hair coloring composition.

14. A dyed product which is obtained by being dyed with the dyeing composition according to any one of claims 1 to 13.

15. An azo coloring agent which is represented by Formula 1-A,
A^{1A}-N=N-C^{1A}-N=N-B^{1A} Formula 1-A
wherein, in Formula 1-A,
C^{1A} represents a group represented by any one of Formulae (C-1), (C-2), (C-3), (C-4), and (C-5),
A^{1A} represents an aromatic group or a group represented by any one of Formulae (A-1), (A-9), (A-14), and (A-23),
B^{1A} represents a group represented by any one of Formulae (B-5), (B-6), (B-7), (B-13), (B-14), and (B-15), and
none of A^{1A}, B^{1A}, and C^{1A} has a carboxy group, a sulfo group, -SO₂F, or a cation structure, 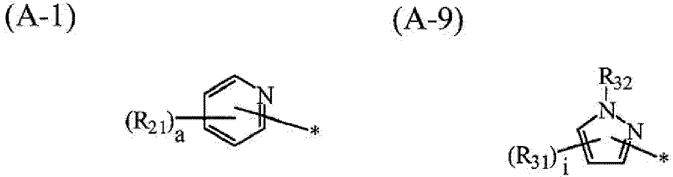 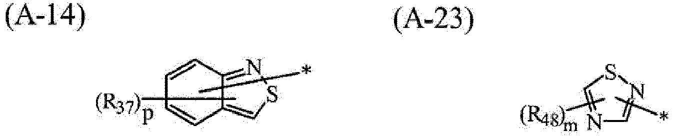
wherein, in Formulae (A-1), (A-9), (A-14), and (A-23),
* represents a position bonded to an azo group in Formula 1-A,
each of R₂₁, R₃₁, R₃₂, R₃₇, and R₄₈ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
adjacent groups among R₂₁, R₃₁, R₃₂, R₃₇, and R₄₈ may be bonded to each other to form a saturated or unsaturated 5- or 6-membered ring structure,
each of a and p independently represents an integer from 0 to 4,
i represents an integer from 0 to 2, and
m represents 0 or 1, 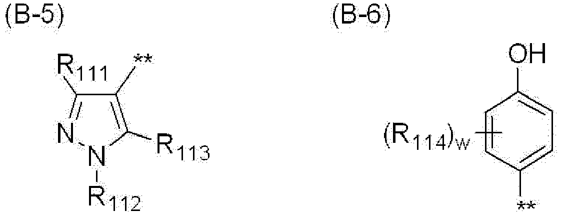 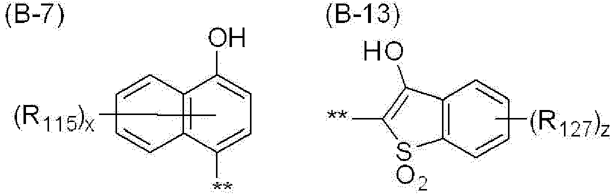 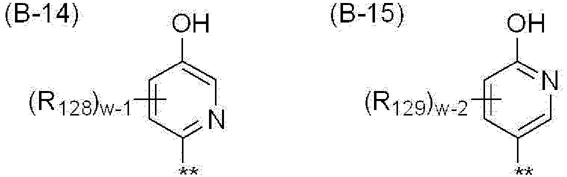
wherein, in Formulae (B-5), (B-6), (B-7), (B-13), (B-14), and (B-15),
** represents a position bonded to an azo group in Formula 1-A,
R₁₁₁ represents an alkyl group, an aryl group, an alkoxy group, an amino group, an alkoxycarbonyl group, a cyano group, an acylamino group, or a carbamoyl group,
R₁₁₂ represents a hydrogen atom, an alkyl group, an aryl group, or a heterocyclic group,
R₁₁₃ represents a hydroxy group or an amino group,
each of R₁₁₄ and R₁₁₅ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w represents an integer from 0 to 4,
x represents an integer from 0 to 6,
each R₁₂₇ independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
z represents an integer from 0 to 4,
each of R₁₂₈ and R₁₂₉ independently represents a halogen atom, an alkyl group, an aryl group, a heterocyclic group, a nitro group, an alkoxy group, an aryloxy group, an amino group, an acylamino group, an alkoxycarbonylamino group, an aminocarbonylamino group, an alkylsulfonylamino group, an arylsulfonylamino group, an alkylthio group, an arylthio group, a heterocyclic thio group, an alkoxycarbonyl group, or a carbamoyl group,
w-1 represents an integer from 0 to 3, and
w-2 represents an integer from 0 to 3, 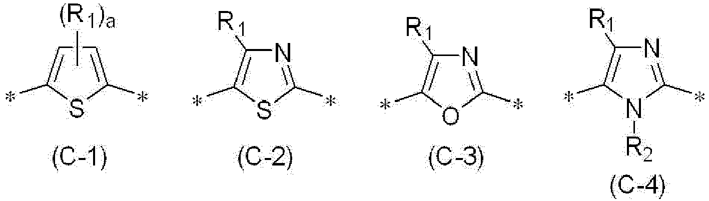 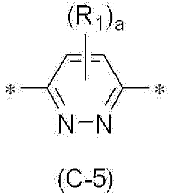
wherein, in Formulae (C-1), (C-2), (C-3), (C-4), and (C-5),
each R₁ independently represents a hydrogen atom, an alkyl group, an aryl group, a heterocyclic group, a cyano group, a hydroxy group, a nitro group, an alkoxy group, an aryloxy group, an amino group, an alkylamino group, a dialkylamino group, an anilino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group,
a represents an integer from 0 to 2,
R₂ represents a hydrogen atom, an alkyl group, or an aryl group, and
* represents a position bonded to an azo group in Formula 1-A.

16. An azo coloring agent which is represented by Formula 2-A,
D^{1A}-N=N-E^{1A} Formula 2-A
wherein, in Formula 2-A,
D^{1A} represents an anthraquinonyl group, and
E^{1A} represents a group represented by Formula E-1, 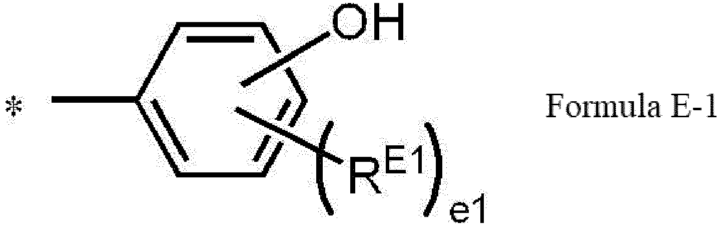
wherein, in Formula E-1,
* represents a position bonded to an azo group in Formula 2-A,
each R^{E1} independently represents a halogen atom, an alkyl group, an aryl group, a heterocyclic group, a cyano group, a hydroxy group, a nitro group, an alkoxy group, an aryloxy group, an amino group, an acylamino group, an aminocarbonylamino group, an alkoxycarbonylamino group, an alkylsulfonylamino group, an arylsulfonylamino group, an alkylthio group, an arylthio group, a heterocyclic thio group, a sulfamoyl group, an alkylsulfonyl group, an arylsulfonyl group, an acyl group, an alkoxycarbonyl group, or a carbamoyl group, and
e1 represents an integer from 1 to 4.
